# EUROPEAN PATENT SPECIFICATION

(11) **EP 0 528 686 B1**
(45) Date of publication and mention of the grant of the patent: **05.11.2003**
(21) Application number: 92307577.4
(22) Date of filing: 12.08.1992
(51) Int. Cl.: C12N 15/62, C12P 21/02, C12N 15/16

(54) **Process for producing peptides**
Verfahren zur Herstellung von Peptiden
Procédé pour la production de peptides

(30) Priority: 19.08.1991 JP 23076991; 31.07.1992 JP 22352092
(43) Date of publication of application: 24.02.1993
(73) Proprietor: Daiichi Suntory Pharma Co., Ltd., Tokyo (JP)
(72) Inventor: Yabuta, Masayuki, Tatebayashi-shi, Gunma (JP); Suzuki, Yuji, Ashikaga-shi, Tochigi (JP); Ohsuye, Kazuhiro, Ohra-gun, Gunma (JP); Oshima, Takehiro, Ashikaga-shi, Tochigi (JP); Onai, Seiko, Isesaki-shi, Gunma (JP); Magota, Koji, Takatsuki-shi, Osaka (JP); Tanaka, Shoji, Ashiya-shi, Hyogo (JP)
(74) Representative: Stoner, Gerard Patrick

(56) References cited:
- EP-A- 0 281 418
- JOURNAL OF BIOCHEMISTRY vol. 102, no. 1 , July 1987 , TOKYO JP pages 111 - 122 SAITO ET AL. 'Bacterial synthesis of recombinant alpha-Human Atrial Natriuretic polypeptide'
- GENE. vol. 32 , 1984 , AMSTERDAM NL pages 321 - 327 SMITH ET AL. 'Chemical synthesis and cloning of a poly(arginine)-coding gene fragment designated to aid polypeptide purification'
- LI ET AL.: 'Characterization of mutants affecting the KRK sequence in the C-terminal domain of lac repressor' JOURNAL OF BIOLOGICAL CHEMISTRY vol. 270, no. 18, 05 May 1995, pages 10640 - 10649
- PARSELL ET AL.: 'The structural stability of a protein is an important determinant of its proteolytic susceptibility in E. coli' JOURNAL OF BIOLOGICAL CHEMISTRY vol. 264, no. 13, 05 May 1989, pages 7590 - 7595
- TAJIMA ET AL.: 'High-level synthesis in E.coli of recombinant human calcitonin: Collagenase claevage of the fusion protein and peptidylglycine alpha-amidation' JOURNAL OF FERMENTATION AND BIOENGINEERING vol. 72, no. 5, 1991, pages 362 - 367

## Description

The present invention relates to a method for producing peptides or their precursors (usually referred to as the "target peptide" in the present text) as fusion proteins.

There have thus far been numerous efforts made to produce physiologically active peptides or proteins in microorganisms such as Escherichia coli using recombinant DNA technology. In such cases, when short peptides having relatively low molecular weights are produced by direct expression systems using microorganisms such as Escherichia coli, the short peptide ends up being rapidly broken down within the microorganism.

In order to inhibit this degradation, a method is used in which, after producing the target peptides in the form of fusion proteins with other proteins or polypeptides (referred to herein as the protective peptides), chemical or enzymatic treatment is performed to specifically release the target peptides from the fusion proteins, followed by separation and purification.

A known method for releasing the target peptide from the fusion protein, in cases where the molecules of the target peptide do not contain a methionine residue, comprises producing a fusion protein in which a methionine residue is introduced at the C-terminal amino acid position of a linker peptide between the protective peptide and the target peptide, followed by cleavage of the methionine residue by treatment with cyanogen bromide (CNBr) to release the target peptide (Science, 198, 1059 (1977), Proc. Natl. Acad. Sci., 76, 106 (1978).

Where the molecule of the target peptide does not contain any arginine or lysine residue, after producing a fusion protein in which an arginine residue or lysine residue is introduced at the C-terminal amino acid position of a linker peptide between a protective peptide and the target peptide, a method used to release the target peptide comprises treatment with trypsin which specifically cleaves at the C-terminal of arginine or lysine residues (Nature, 285, 456 (1980), or treatment with lysyl endopeptidase (Achromobacter protease I) which specifically cleaves at the C-terminal of a lysine residue.

A protein fragment originating in the host microorganism used in production is commonly used as the protective peptide to produce the fusion protein. A polypeptide of a suitable size (length) from the N-terminal (amino terminal region) of a protein expressed in large amounts in the cells of the host microorganism is used, and it is known that the length of that polypeptide has a considerable influence on the productivity of the fusion protein.

For example, although one might expect to improve productivity by reducing the size (shortening the length) of the protective peptide region, to increase the relative proportion of target protein with respect to said fusion protein, reduction of said protective peptide region does not in fact always improve productivity of the target peptide. For example, when insulin was produced in Escherichia coli as a fusion protein, although the productivity of insulin increased when β-galactosidase was used as the protective peptide and the size of the β-galactosidase portion was reduced, it is also known that insulin productivity decreases with further reduction of the size (Gene, 29, 251, 1984).

When producing a target peptide as a portion of a fusion protein, there is no established theory as to how large the protective peptide should be despite the size of the protective peptide being closely related to the stability of the fusion protein inside the microorganism. In general, stable fusion proteins form insoluble inclusion bodies in host cells. In other words, in order to produce a large amount of fusion protein it is advantageous to form inclusion bodies in host cells.

However, depending on the particular case, when the number of inclusion bodies expressed per cell is increased the inclusion bodies cause damage to the cells, resulting in inhibition of cell growth which may decrease the productivity of inclusion bodies per volume of culture. Although the mechanism of formation of inclusion bodies in microorganisms is described in detail in the paper by Catherine (Biotechnology, 7, 1141 (1989)), numerous factors are involved in inclusion body formation and there is no established theory at present. Nor are there yet any established methods for production on an industrial scale by expressing a fusion protein as stable inclusion bodies in microorganisms.

There are numerous reports of methods in which human calcitonin is produced as a fusion protein in Escherichia coli. For example, Bennett et al. reported a method for producing human calcitonin precursor (hCT-Gly) by expressing a fusion protein of chloramphenicol acetyltransferase and human calcitonin precursor (hCT-Gly) (JP-A-60/501391).

However, this method has a low level of efficiency with only 1.1-2.0 mg of human calcitonin precursor obtained from 44 mg of fusion protein.

Our EP-A-281418 (corresponding to JP-A-1/010999) describes an efficient method for producing target peptides in E.coli using a fusion protein of E.coli β- galactosidase and the target peptide (specifically human calcitonin precursor hCT-Gly-Lys-Lys-Arg). The fusion protein A-L-B contains the target peptide B, a linker amino acid residue L, preferably lysine, arginine, glutamic acid or methionine, and a protective "partner" polypeptide A which is the β-galactosidase sequence. The fusion protein is expressed as inclusion bodies which are obtained as an insoluble fraction after disrupting the cultured cells, solubilized and then treated with endoprotease or other suitable chemical agent to liberate the target peptide from the fusion protein.

In another of our disclosures relating to the production of human calcitonin, after solubilizing the fusion protein (as described above) with urea, human calcitonin precursor (hCT-Gly-Lys-Lys-Arg) was released from the fusion protein with V8 protease, hCT-Gly was obtained by removing Lys-Lys-Arg of the C-terminal portion of human calcitonin precursor using carboxypeptidase B, and the mature C-terminal amidated human calcitonin was obtained with high efficiency using a Xenopus laevis C-terminal amidation enzyme (JP-A-2/190193).

However, there is still a need for an efficient method for producing peptide.

The present invention aims to provide a novel method for producing peptides, such as physiologically active peptides or precursors thereof, preferably in large amounts.

As a result of the inventors of the present invention studying a means of solving the above-mentioned objects, it was experimentally verified for the first time that in order to improve the productivity of fusion protein in Escherichia coli cells, the selection of a fusion protein isoelectric point within the range of pI 4.9 to pI 6.9 can improve stability and productivity of said fusion protein. In addition, completely new findings were obtained wherein the above-mentioned objects are solved by regulating the number of amino acids having a charge within the linker peptide (basic or acidic amino acid residues) to adjust the isoelectric point of the fusion protein to within a range of pI 4.9 to pI 6.9.

Aspects of the present invention are set out in the independent claims 1 and 10.

The invention relates to the production of a target peptide selected from calcitonin, calcitonin precursor, natriuretic peptide (NP), cell growth factor and parathyroid hormone, comprising:
(a) culturing host cells transformed with vector able to express a gene coding for a fusion protein representable by the formula

   A-L-B

   in which
   B is the target peptide;
   A is a protective peptide, being a 90 to 210 amino acid N-terminal fragment of E. coli β-galactosidase, and
   L is a linker peptide between the C-terminal of the protective peptide and the N-terminal of the target peptide,
   the linker peptide L being selected so that the target protein separates off when the fusion protein is treated with a predetermined chemical substance such as an enzyme;
(b) homogenising the cultured host cells to obtain an insoluble fraction comprising inclusion bodies;
(c) solubilizing the fusion protein in the inclusion bodies by treating the insoluble fraction with solubilizing agent, and
(d) cleaving the peptide bond between the C-terminal amino acid residue of the linker and the N-terminal of the target peptide to release the target peptide.

We propose selecting the linker peptide L so as to give the fusion protein an isoelectric point in the range 4.9 to 6.9. We find that this can improve productivity.

In a first aspect (claim 1) the linker peptide L is all or part of the amino acid sequence shown in SEQ ID NO: 1 and contains from 3 to 8 arginine residues.

A second aspect (claim 10) specifies that the linker peptide L, selected so as to achieve the specified isoelectric point in the fusion protein, is not a single lysine, arginine, glutamic acid or methionine residue as suggested in our EP-A-281418.

### Brief Description of the Drawings

Fig. 1 indicates the nucleotide sequence of a portion of the tetracycline resistant gene of nucleotide positions 403-495 within plasmid pBR322, the corresponding amino acid sequence and the portions coding for each linker peptide. This nucleotide sequence is indicated in SEQ ID NO 1.
Fig. 2 indicates a process for construction of plasmids pG97S4DhCT(G)R10 and pG97S4DhCT(G)R6.
Fig. 3 indicates a process for construction of plasmid pG97S4DhCT(G)R8.
Fig. 4 indicates a process for construction of plasmids pG97S4DhCT(G)R1-R5.
Fig. 5 indicates the nucleotide sequences of R1 through R5 which are DNA coding for the linker portions. These sequences are also indicated in SEQ ID NOS 2 to 9.
Fig. 6 indicates the amino acid sequence of each linker peptide.
Fig. 7 is a photograph of SDS polyacrylamide gel electrophoresis indicating the states of expression of the fusion proteins from each of the expression plasmids.
Fig. 8 indicates the results of high performance liquid chromatography of human calcitonin obtained by a method of the present invention.
Fig. 9 indicates the amino acid sequence of CNP-22, the nucleotide sequence coding for it, and the PCR primers for insertion of restriction enzyme sites to both ends.
Fig. 10 indicates a process for construction of plasmid pG97S4DhCNP-22.
Fig. 11 indicates a linker peptide for adjusting the isoelectric point of a fusion protein and an oligonucleotide sequence coding for it.
Fig. 12 indicates a process for construction of plasmids pG97S4DhCNP-22R3-2 and pG97S4DhCNP-22R5-2.
Fig. 13 indicates a process for construction of plasmid pG97S4DhCNP-22R5-3.
Fig. 14 is a photograph of SDS polyacrylamide gell electrophoresis indicating a result comparing the amounts of fusion proteins expressed for which the isoelectric points have been adjusted.
Fig. 15 indicates the elution profile of fusion protein before release of hCNP-22 in high performance liquid chromatography.
Fig. 16 indicates the elution profile of fusion protein after release of hCNP-22 in high performance liquid chromatography.

The method of the present invention is applicable for production of fusion proteins of physiologically active target peptides and particularly to their production via inclusion bodies. In addition to human calcitonin explained in detail in the present specification, examples of such peptides include non-human calcitonins and precursors thereof, natriuretic peptides (NP) such as atrial natriuretic peptide, brain natriuretic peptide or C-type natriuretic peptide, cell growth factors and parathyroid hormone (PTH).

Particularly preferable protective peptides are peptides consisting of the 1st to 97th amino acids of the N-terminal of Escherichia coli β-galactosidase. It is even more preferable to use peptides wherein the cysteine residue is substituted by a serine residue in peptide consisting of these 97 amino acid residues. Furthermore, it is most preferable to use a peptide wherein the cysteine residue is replaced by a serine residue in a peptide consisting of the above-mentioned 97 amino acid residues, and moreover, four glutamic acid residues are replaced by aspartic acid residues.

The present invention is characterized by the production of the target peptide in the form of a fusion protein having an isoelectric point between 4.9 and 6.9. In this case, the isoelectric point of the fusion protein can be determined in the following manner from the amino acid sequence. A suitable method for calculating the isoelectric point is in accordance with the method described in Trends in Analytical Chemistry, Vol. 5, No. 4, pp. 82-83 (1986). For example, the isoelectric point estimation program DNASIS (Hitachi) prepared based on this method can be used.

In order to regulate the isoelectric point of the fusion protein within the above-mentioned range, the amino acid residues of the protective peptide and/or linker peptide must be regulated, since the amino acids of the target peptide cannot be altered. A natural peptide or portion of such peptide that gives the above-mentioned isoelectric point can be selected for the protective peptide and/or linker peptide to form a fusion peptide with the target peptide. Another method involves regulation of the isoelectric point of the fusion protein by substitution, elimination or addition of amino acid residues of natural peptides or portions of those peptides.

The addition or elimination of acidic amino acids such as aspartic acid and glutamic acid residues, the addition or elimination of basic amino acids such as arginine and lysine residues, substitution of other amino acid by such amino acids, or combinations of such amino acid manipulations can be used for this regulation.

As mentioned above, regulation of the isoelectric point of the fusion protein is performed with the linker peptide.

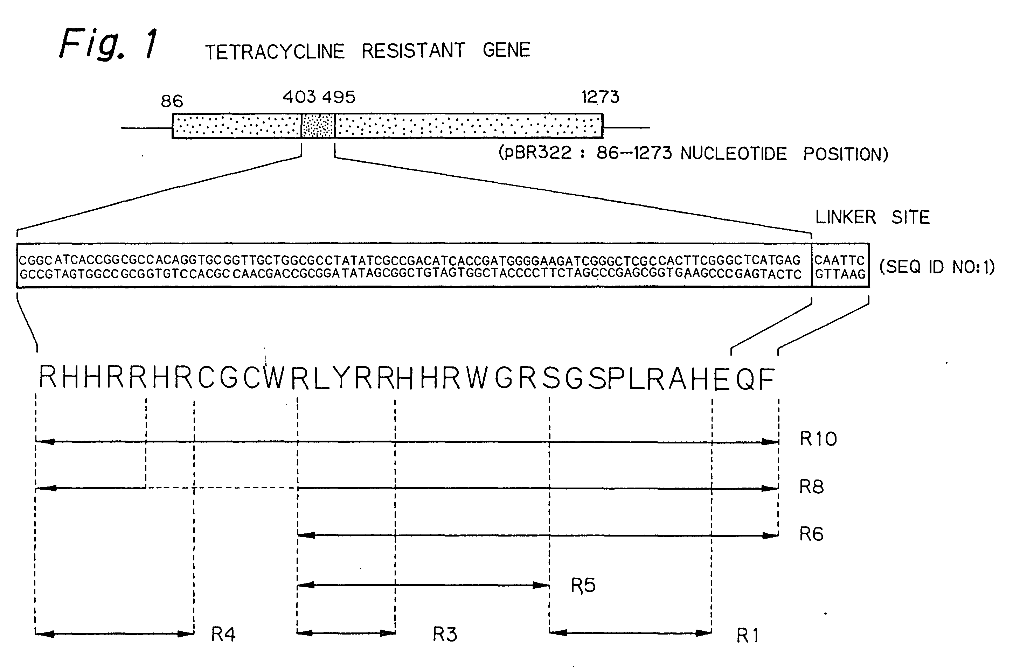

A peptide or a portion thereof that can be coded by the 403rd to 495th nucleotide sequence of the tetracycline-resistance gene derived from pBR322 can be used for this type of peptide. As is indicated in Fig. 1 and SEQ ID NO 1, this gene region can code for 10 arginine residues among 33 amino acid residues, and by using the various portions in this region, various linker peptides can be obtained containing different numbers of arginine residues. In this case, it is preferable to use a portion containing 1 or more, preferably 3 or more and particularly preferably 3 to 8 arginine residues.

In order to release a target peptide from a fusion protein, it is necessary that an amino acid residue that can be enzymatically or chemically cleaved be present at the C-terminal of the linker peptide. Examples of such amino acid residues that are usable include arginine residue or lysine residue (cleaved by trypsin), a lysine residue (cleaved by lysyl endopeptidase), glutamine residue (cleaved by V8 protease), and methionine residue (cleaved with cyanogen bromide).

There now follows an explanation of production of a plasmid that expresses a fusion protein, taking an example in which the target peptide is human calcitonin precursor.

Plasmid pG97S4DhCT(G) is used as a starting plasmid for constructing expression plasmids for a expressing human calcitonin precursor comprising the amino acid sequence of human calcitonin and a glycine residue added to its C-terminal in form of fusion proteins containing various linkers.

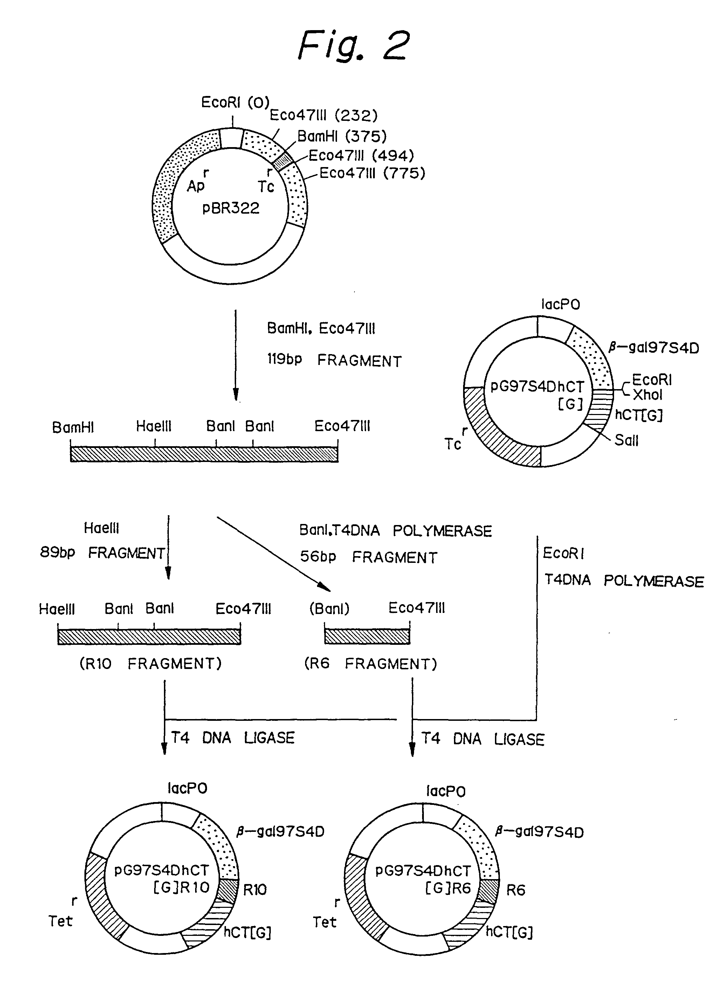

In this plasmid, (a) the structural gene (β-ga197S4D) coding for a protective peptide consisting of 97 amino acids of the N-terminal of Escherichia coli β-galactosidase, wherein a cysteine residue contained therein is substituted by a serine residue and four glutamic acid residues are substituted by aspartic acid residues, and (b) a structural gene of human calcitonin precursor, are linked via the EcoRI and XhoI recognition sites as indicated in Fig. 2. The structural gene coding for this fusion protein is under the control of the lac promoter. Moreover, this plasmid contains a gene for a selection marker.

This plasmid is derived from pG97SHPCTLE(G) plasmid described in JP-A-2/190193.

Escherichia coli W3110 containing the above-mentioned pG97S4DhCT(G) plasmid was deposited to the Fermentation Research Institute, Agency of Industrial Science and Technology, as Escherichia coli SBM323 on August 8, 1991 based on the provisions of the Budapest treaty, and given FERM BP-3503.

A fusion protein encoded by pG97S4DhCT(G) plasmid comprises a protective peptide consisting of 97 amino acids of the end of the N-terminal of β-galactosidase, in which the cysteine residue contained therein is substituted by a serine residue, and 4 glutamic acid residues are substituted by aspartic acid residues, and the above-mentioned human calcitonin precursor. Its expression is extremely low, and its isoelectric point is 4.43. Thus, in order to express various fusion proteins over a broad range of isoelectric points, DNA sequences coding for linker peptides having various basic amino acid residues are inserted between the above-mentioned β-galactosidase gene and human calcitonin precursor gene using the EcoRI-XhoI site.

It is convenient to use as linker peptide a peptide comprising 33 amino acids and able to be encoded by a gene divided from the tetracycline-resistance gene of nucleotide positions 86-1273 in plasmid pBR322, or a portion of said peptide. Ten arginine residues are distributed in this peptide region, and by using suitable portions for the linker peptide, fusion proteins can be obtained having various isoelectric points.

The linker peptides obtained from this region along with the nucleotide sequences that code for such linker peptides are indicated in Fig. 1. The genes that code for these peptides are obtained by digestion of plasmid pBR322 with a suitable restriction enzyme, or by chemical synthesis in accordance with a commonly used method. These linker peptides R₁, R₃, R₄, R₅, R₆, R₈ and R₁₀ contain 1, 3, 4, 5, 6, 8 and 10 arginine residues, respectively. The expression plasmids in which a gene cloning for one of these linker peptides is inserted at the EcoRI-XhoI site in the above-mentioned plasmid pG97S4DhCT(G) are referred to as pG97S4DhCT(G)R1, pG97S4DhCT(G)R3, pG97S4DhCT(G)R4, pG97S4DhCT(G)R5, pG97S4DhCT(G)R6, pG97S4DhCT(G)R8 and pG97S4DhCT(G)R10.

In addition, Escherichia coli W3110 strains obtained by transformation with the above-mentioned starting plasmid and these plasmids are referred to as W3110/pG97S4DhCT(G), W3110/pG97S4DhCT(G)Rl, W3110/RG97S4DhCT(G)R3, W3110/pG97S4DhCT(G)R4, W3110/pG97S4DhCT(G)R5, W3110/pG97S4DhCT(G)R6, W3110/pG97S4DhCT(G)R8 and W3110/pG97S4DhCT(G)R10.

The following results are obtained when the isoelectric points of the fusion proteins produced by these microorganisms are calculated:
W3110/pG97S4DhCT(G) (the number of arginine residues in linker region = 0); isoelectric point = 4.43,
W3110/pG97S4DhCT(G)R1 (the number of arginine residues in linker region = 1); isoelectric point = 4.70,
W3110/pG97S4DhCT(G)R3 (the number of arginine residues in linker region = 3); isoelectric point = 4.90,
W3110/pG97S4DhCT(G)R4 (the number of arginine residues in linker region = 4); isoelectric point = 5.80,
W3110/pG97S4DhCT(G)R5 (the number of arginine residues in linker region = 5); isoelectric point = 5.91,
W3110/pG97S4DhCT(G)R6 (the number of arginine residues in linker region = 6); isoelectric point = 6.01,
W3110/pG97S4DhCT(G)R8 (the number of arginine residues in linker region = 8); isoelectric point = 6.83, and
W3110/pG97S4DhCT(G)R10 (the number of arginine residues in linker region = 10); isoelectric point = 7.85.

Escherichia coli transformants producing the above-mentioned fusion protein having an isoelectric point from 4.43 to 7.85 were cultured, and analyzed for the amount of fusion protein produced per number of cells by SDS polyacrylamide gel electrophoresis. Strain W3110/pG97S4DhCT(G) having an isoelectric point of 4.43 and strain W3110/pG97S4DhCT(G)R1 having an isoelectric point of 4.70 produced only a small amount of fusion protein. It was also verified that the other strains produced a large amount of fusion protein as inclusion bodies in bacterial cells. Thus, it was indicated by these results that if the isoelectric point of the fusion protein is in the vicinity of 4.5-4.7, both the productivity of fusion protein and the amount of inclusion bodies produced are poor.

Moreover, although the productivity of fusion protein per number of cells in the case of strain W3110/pG97S4DhCT(G)R10 having an isoelectric point of 7.85 was roughly equivalent to that of the other bacterial strains, it was verified that the final bacterial concentration during culturing was only half that of other strains. It is believed that, although the capacity to form fusion protein per number of cells increased, with the weakly alkaline isoelectric point of the fusion protein (pI 7.85) inhibition of cell growth occurred because of rapid formation of inclusion bodies, or because of the isoelectric point of the fusion protein being weakly alkaline, thus resulting in an absence of increase in final bacterial concentration.

Based on the above results, it was verified for the first time by the inventors of the present invention that in order to produce fusion protein both stably and in a large amount within Escherichia coli cells in the form of inclusion bodies, it is important that the isoelectric point of the fusion protein be within the acidic pI between 4.9 and 6.9.

Next, we performed separation and purification of human calcitonin precursor (hCT-Gly) from inclusion bodies of fusion protein of human calcitonin precursor produced in Escherichia coli in the manner described above. Moreover, it was verified that human calcitonin having the amidated C-terminal can be efficiently produced using amidation enzyme derived from Xenopus laevis.

The following provides another example of the present invention for construction of a plasmid that expresses a fusion protein taking as an example the case wherein the target peptide is human C-type natriuretic peptide-22 (to be abbreviated as CNP-22) (SEQ ID NO. 10). Plasmid pG97S4DhCNP-22 was used as a starting plasmid for construction of expression plasmids for expressing human CNP-22 (comprising 22 amino acids) as fusion proteins containing various linkers.

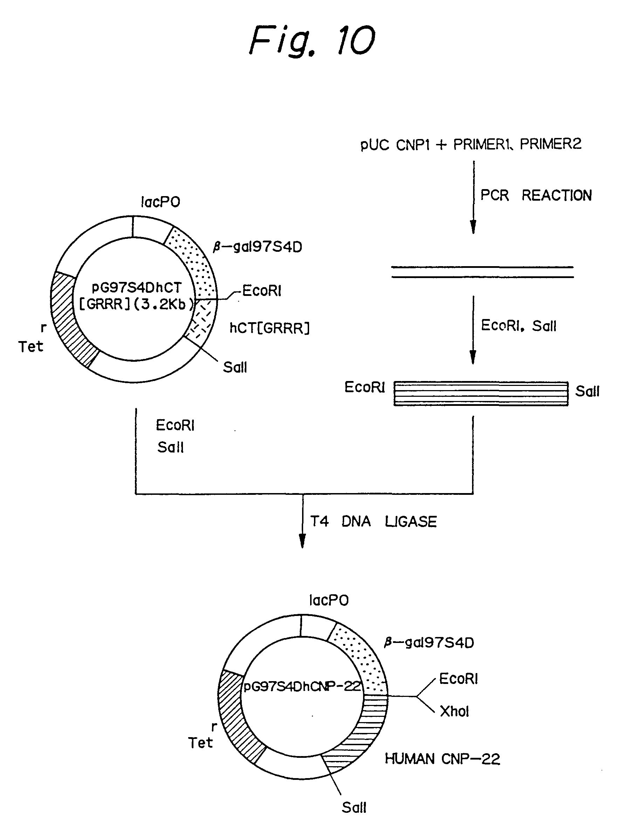

In this plasmid, (a) the structural gene (βgal97S4D) coding for a protective peptide consisting of 97 amino acids of the N-terminal of Escherichia coli β-galactosidase wherein a cysteine residue contained therein is substituted by a serine residue and four glutamic acid residues are substituted by aspartic acid residues, and (b) the structural gene of human CNP-22, are linked via the EcoRI and XhoI recognition sites as indicated in Fig. 10. The structural gene coding for this fusion protein is under the control of the lac promoter. Moreover, this plasmid contains a marker for tetracycline resistance.

In the construction of the pG97S4DhCNP-22 expression plasmid, a human CNP-22 structural gene and the plasmid pUCCNPl used for preparation of said gene are disclosed in JP-A-4/139199.

In addition, plasmid pG97S4DhCT(GRRR) used as a plasmid which codes for a protective peptide is essentially identical to the above-mentioned plasmid pG97S4DhCT(G).

The isoelectric point of the fusion protein encoded by plasmid pG97S4DhCNP-22 is 4.56. From the viewpoint that the isoelectric point of the fusion protein for high expression of fusion protein indicated above is preferably within the range of pI = 4.9 to pI = 6.9, production of a large amount of fusion protein cannot be expected. As such, a study was conducted wherein linker peptide genes coding for various basic amino acids were inserted into the EcoRI-XhoI region between the above-mentioned β-galactosidase gene and human CNP gene for the purpose of designing various fusion proteins having isoelectric points from 4.9 to 6.9 and expressing a large amount of said fusion proteins in Escherichia coli.

These linker peptides can be produced using methods wherein genes coding for basic amino acids are artificially designed and then chemically synthesed.

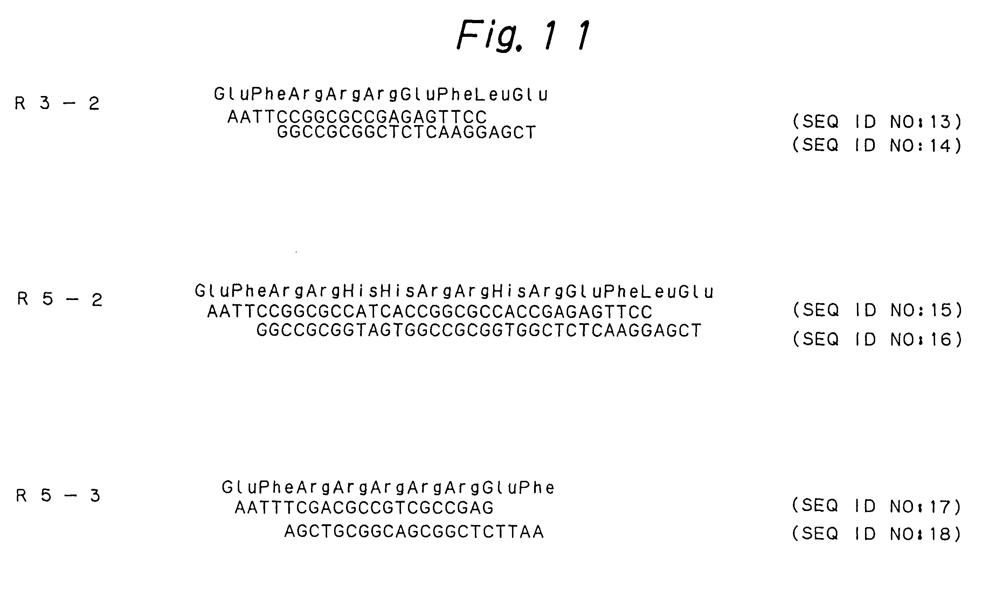

Fig. 11 indicates the design of linker peptide genes coding for basic amino acids and the chemically synthesized gene sequences. These linker peptides, R3-2 (SEQ ID NOs 13 and 14), R5-2 (SEQ ID NOs 15 and 16), and R5-3 (SEQ ID NOs 17 and 18) contain 3, 5 and 5 arginine residues, respectively. The expression plasmids wherein genes coding for these linker peptides have been inserted into the EcoRI-XhoI region in the above-mentioned pG97S4DhCNP-22 are referred to as pG97S4DhCNP-22R3-2, pG97S4DhCNP-22R5-2 and pG97S4DhCNP-22R5-3, respectively.

In addition, Escherichia coli W3110 strains obtained by transformation with the above-mentioned starting plasmids and these plasmids are referred to as W3110/pG97S4DhCN-22, W3110/pG97S4DhCNP-22R3-2, W3110/pG97S4DhCNP-22R5-2, and W3110/pG97S4DhCNP-22R5-3, respectively.

The following results are obtained when the isoelectric points of the fusion proteins produced by these microorganisms are calculated:
W3110/pG97S4DhCNP-22 (the number of arginine residues in linker region = 0); isoelectric point = 4.56,
W3110/pG97S4DhCNP-22R3-2 (the number of arginine residues in linker region = 3); isoelectric point = 4.95,
W3110/pG97S4DhCNP-22R5-2 (the number of arginine residues in linker region = 5); isoelectric point = 6.22, and
W3110/pG97S4DhCNP-22R5-3 (the number of arginine residues in linker region = 5); isoelectric point = 5.59.

When the Escherichia coli strains producing the above-mentioned fusion protein having isoelectric points from pI 4.56 to pI 6.22 were cultured, and analyzed for the amount of fusion protein produced per number of cells by SDS polyacrylamide gel electrophoresis, although the amount of fusion protein produced by strain W3110/pG97S4DhCNP-22 (isoelectric point of fusion protein = 4.56) was not that high, in the case of those strains that demonstrated fusion protein isoelectric points from pI 4.95 to pI 6.22, it was verified that the amount of fusion protein expressed was large in comparison to strain W3110/pG97S4DhCNP-22.

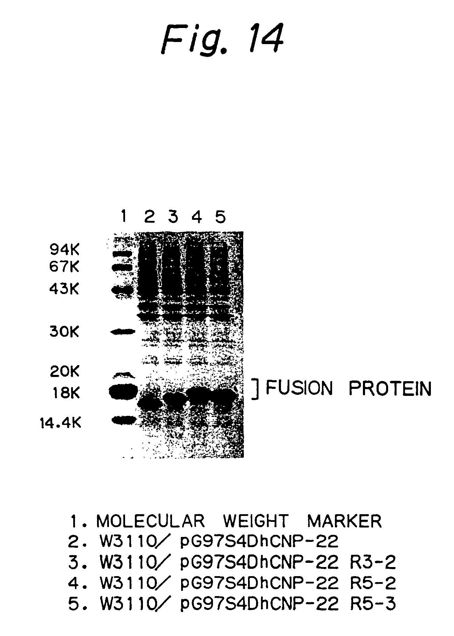

In particular, it was clear that a large amount of fusion protein was expressed by strain W3110/pG97S4DhCNP-22R5-2 providing an isoelectric point of 5.59 and strain W3110/pG97S4DhCNP-22R5-3 providing an isoelectric point of 6.22 in comparison to strain W3110/pG97S4DhDCNP-22 (see Fig. 14). So, the case of human CNP-22 also indicated that processing with the isoelectric point of the fusion protein within the range of pI 4.9-6.9 results in improved productivity of fusion protein.

Moreover, we verified that human CNP-22 from inclusion bodies of fusion protein produced in Escherichia coli in this manner is efficiently released using V8 protease resulting in efficient production of human CNP-22, thereby indicating the usefulness of the present techniques.

### Examples

The following Examples provides a detailed explanation of embodiments.

### Example 1: Construction of Expression Vector

An expression plasmid that expresses fusion protein having 1 to 10 arginine residues in the linker peptide region was constructed in the manner described below.

### (A) Construction of pG97S4DhCT(G)R10

The procedure described below was performed to insert a gene coding for the amino acids of the R10 region indicated in Fig. 1 into the linker peptide coding region between a β-gal 97S4D (peptide consisting 97 amino acids of the N-terminal of β-galactosidase wherein a cysteine residue is substituted by a serine residue and four glutamic acid residues are substituted by aspartic acid residues) gene and an hCT(G) (human calcitonin precursor having 1 glycine residue at the C-terminal) gene. A construction process for pG97S4DhCT(G)R10 is indicated in Fig. 2.

First of all, pBR322 was digested with restriction enzymes to isolate the R10 region within the tetracycline-resistant gene of pBR322. 150 µg of pBR322 was digested with 200 units each of BamHI and Eco47III for 60 minutes at 37°C in 300 µl of High buffer (10 mM Tris/HCl pH 7.5, 100 mM NaCl, 10 mM MgCl₂ and 1 mM dithiothreitol abbreviated as DTT).

After reaction, 30 µl of a dye solution (0.25% bromphenol blue, 0.25% xylene cyanol, 40% sucrose) was added to the reaction mixture, and 1.5% agarose gel electrophoresis (TAE buffer solution; 40 mM Tris-acetic acid pH 8.0, 2 mM EDTA, 120V, 2 hours) was performed. Following electrophoresis, the gel was immersed in 0.5 µg/ml of ethidium bromide solution, the 119 bp of BamHI-Eco47III fragment was cut out from the gel.

The cut out gel was then place in a dialysis tube containing TAE buffer and electrophoresed (120 V, 30 minutes) to elute the 119 bp DNA fragment from the gel. The solution in the dialysis tube was then collected followed by phenol treatment, chloroform treatment and ethanol precipitation according to commonly used methods, and the ethanol precipitate was dissolved in 40 µl of TE buffer (10 mM Tris/HCl pH 8.0, 1 mM EDTA). 2.5 µl of 10-fold concentrated Med buffer (100 mM Tris/HCl pH 7.5, 500 mM NaCl, 100 mM MgCl₂ and 10 mM DTT) and 10 units of HaeIII were added to 20 µl of this BamHI-Eco47III (119 bp) DNA fragment-containing solution, and digested for 2 hours at 37°C. After addition of dye solution, 15% polyacrylamide gel electrophoresis (TBE buffer; 89 mM Tris-boric acid buffer pH 8.0, 2 mM EDTA, 120 V, 90 minutes) was carried out.

And then the gel was stained in ethidium bromide solution, and the band of the HaeIII-Eco47III DNA fragment (89 bp) was cut out from the gel. This cut out gel was cut up into fine pieces and allowed to stand for 12 hours at 37°C in 200 µl of DNA elution buffer (0.5 M ammonium acetate, 1 mM EDTA pH 8.0). Phenol treatment, chloroform treatment and ethanol precipitation were then performed according to commonly used methods, and the ethanol precipitate was dissolved in 5 µl of TE buffer (10 mM Tris/HCl pH 8.0, 1 mM EDTA).

Next, 5 µg of pG97S4DhCT(G) plasmid was digested for 2 hours at 37°C in 50 µl of TA buffer (33 mM Tris/acetic acid pH 7.9, 10 mM magnesium acetate, 0.5 mM DTT, 66 mM potassium acetate, 0.01% bovine serum albumin) with 30 units of EcoRI. Moreover, 1 µl of 25 mM dNTP consisting of dATP, dGTP, dCTP and dTTP and 4 units of T4 DNA polymerase were added to this reaction solution to fill in the cohesive ends for 5 minutes at 37°C. After the reaction, phenol treatment, chloroform treatment and ethanol precipitation were performed according to commonly used methods, and the ethanol precipitate was dissolved in 10 µl of TE buffer.

Five µl of EcoRI-digested and blunt ended pG97S4DhCT(G) and 5 µl of the HaeIII-Eco47III DNA fragment (89 bp) were mixed, and ligation reaction was carried out for 12 hours at 16°C with a DNA ligation kit (Takara Shuzo). The ligation mixture was used to transform Escherichia coli W3110 according to commonly used methods, and tetracycline-resistant transformants were obtained. The plasmid structure was confirmed by restriction enzyme analysis with BspHI and BglII, and one of the transformants carring a desired structure was named W3110/pG97S4DhCT(G)R10 strain.

### (B) Construction of pG97S4DhCT(G)R6

The procedure described below was performed to insert a gene coding for the amino acids of the R6 region indicated in Fig. 1 into the linker peptide-coding between β-gal 97S4D gene. The construction of pG97S4DhCT(G)R6 is shown in Fig. 2.

pBR322 was digested with restriction enzymes in order to isolate the R6 region within the tetracycline-resistant gene of pBR322. 150 µg of pBR322 was digested with 200 units each of BamHI and Eco47III for 60 minutes at 37°C in 300 µl of High buffer (10 mM Tris/HCl pH 7.5, 100 mM NaCl, 10 mM MgCl₂ and 1 mM DTT). After the reaction, 30 µl of a dye solution (0.25% bromphenol blue, 0.25% xylene cyanol, 40% sucrose) was added to the reaction mixture, and the reaction mixture was used for 1.5% agarose gel electrophoresis (TAE buffer solution; 40 mM Tris-acetic acid pH 8.0, 2 mM EDTA, 120V, 2 hours).

After the gel electrophoresis, the gel was stained in 0.5 µg/ml of ethidium bromide solution, and the band of the BamHI-Eco47III (119 bp) DNA fragment was cut out from the gel. The cut out gel was then place in a dialysis tube containing TAE buffer and electrophoresed (120 V, 30 minutes) to elute the 119 bp DNA fragment from the gel. The solution in the dialysis tube was then collected followed by phenol treatment, chloroform treatment and ethanol precipitation according to commonly used methods, and the ethanol precipitate was dissolved in 40 µl of TE buffer (10 mM Tris/HCl pH 8.0, 1 mM EDTA).

2.5 µl of 10-fold concentrated TA buffer (330 mM Tris/HCl pH 7.9, 100 mM magnesium acetate, 5 mM DTT, 660 mM potassium acetate and 0.1% bovine serum albumin) and 10 units of BanI were added to 20 µl of this BamHI-Eco47III (119 bp)-containing solution and incubated for 2 hours at 37°C. Furthermore, 1 µl of 25 mM dNTP and 4 units of T4 DNA polymerase were added to fill in the cohesive ends for 5 minutes at 37°C. After addition of 2.5 µl of dye solution to the reaction mixture, 15% polyacrylamide gel electrophoresis (TBE buffer; 89 mM Tris-boric acid buffer pH 8.0, 2 mM EDTA, 120 V, 90 minutes) was carried out.

Following the electrophoresis, the gel was stained in ethidium bromide solution, and the band of the HaeIII-Eco47III DNA fragment (56 bp) was cut out from the gel. This cut out gel was cut up into fine pieces and allowed to stand for 12 hours at 37°C in 200 µl of DNA elution buffer (0.5 M ammonium acetate, 1 mM EDTA pH 8.0). Phenol treatment, chloroform treatment and ethanol precipitation were then performed according to commonly used methods, and the ethanol precipitate was dissolved in 5 µl of TE buffer (10 mM Tris/HCl pH 8.0, 1 mM EDTA).

5 µg of pG97S4DhCT(G) plasmid was digested with 30 units of EcoRI for 2 hours at 37°C in 50 µl of TA buffer. Moreover, 1 µl of 25 mM dNTP and 4 units of T4 DNA polymerase were added to this reaction solution fill in the cohesive ends for 5 minutes at 37°C. After the reaction, phenol treatment, chloroform treatment and ethanol precipitation were performed according to commonly used methods, and the ethanol precipitate was dissolved in 10 µl of TE buffer.

5 µl of EcoRI-digested and blunt ended pG97S4DhCT(G) and 5 µl of the BanI-Eco47III DNA fragment (56 bp) were mixed, and ligation reaction was performed for 12 hours at 16°C with a DNA ligation kit (Takara Shuzo). The reaction mixture was used to transform Escherichia coli W3110 according to commonly used methods, and tetracycline-resistant transformants were obtained. The plasmid structure was confirmed by restriction enzyme analysis with BspHI and BglII, and one of the transformants carring a desired structrure was named W3110/pG97S4DhCT(G)R6.

### (C) Construction of pG97S4DhCT(G)R8

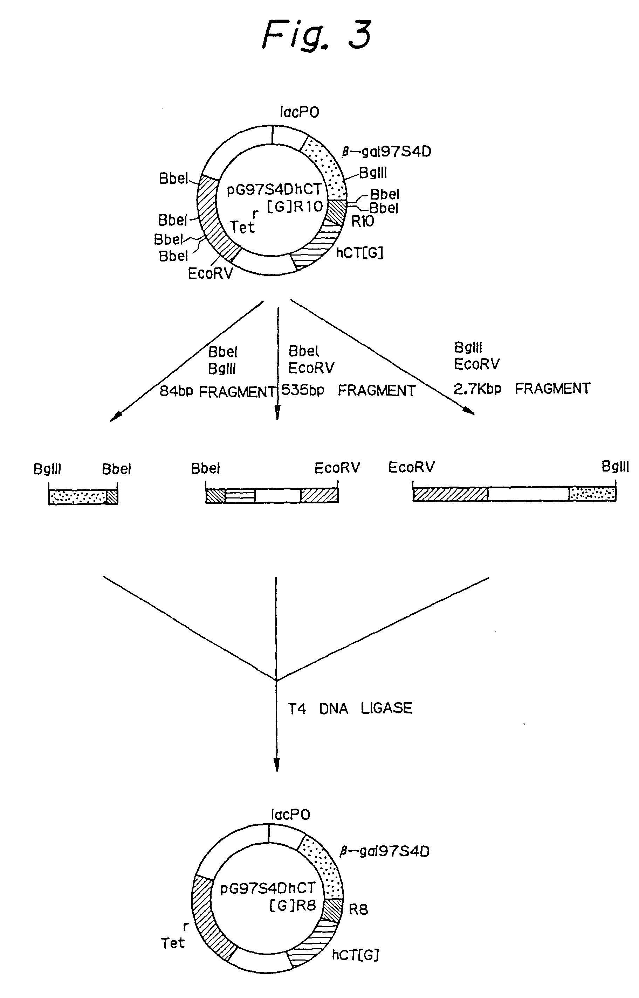

The procedure described below was performed to insert a gene coding for the amino acids of the R8 region indicated in Fig. 1 into the linker peptide cording region between β-gal 97S4D gene and an hCT(G) gene. The construction of pG97S4DhCT(G)R8 is indicated in Fig. 3.

Three tubes were prepared by placing 10 µg of pG97S4DhCT(G)R10 into each tube containing 50 µl of High buffer. 20 units of BbeI and BglII, BbeI and EcoRV, and BglII and EcoRV were respectively added in the three tubes. After allowing to react for 2 hours at 37°C, 1.5% agarose gel electrophoresis was performed, and the BbeI-BglII (84 bp), BbeI-EcoRV (353 bp) and BglII-EcoRV (2.7 Kb) and bands were cut out from the gel, and electrophoresis was carried out.

Phenol treatment, chloroform treatment and ethanol precipitation were then performed according to commonly used methods, after which the ethanol precipitate was dissolved in 5 µl of TE buffer. The three DNA fragments obtained in this manner were mixed and ligated for 12 hours at 16°C using a DNA ligation kit. The ligation mixture was used to transform Escherichia coli W3110 according to commonly used methods, and tetracycline-resistant transformants were obtained. The plasmid structure was confirmed by restriction enzyme analysis, and one of the transformant was named E. coli W3110/pG97S4DhCT(G)R8 strain.

### (D) Construction of pG97S4DhCT(G)R1, pG97S4DhCT(G)R3, pG97S4DhCT(G)R4 and pG97S4DhCT(G)R5

The procedure described below was performed to insert gene coding for the amino acids of the R1, R3, R4 or R5 region indicated in Fig. 1 into the linker peptide-coding region between a β-gal 97S4D gene and an hCT(G) gene.

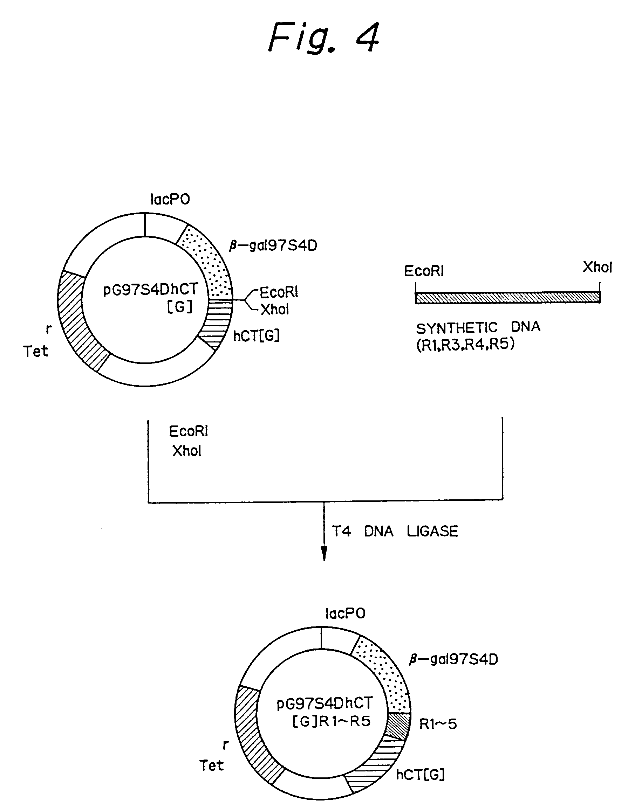

The construction of pG97S4DhCT(G)R1, pG97S4DhCT(G)R3, pG97S4DhCT(G)R4 and pG97S4DhCT(G)R5 is indicated in Fig. 4.

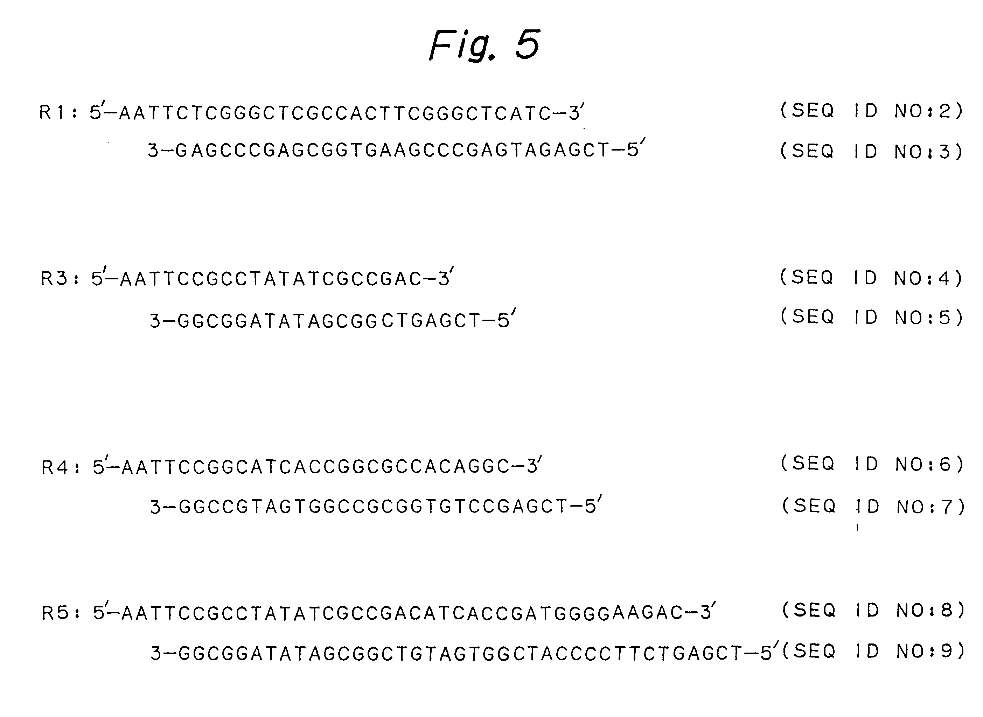

25 µg of pG97S4DhCT(G) was digested with 30 units of XhoI, and with EcoRI, in 50 µl of High buffer for 2 hours at 37°C. Following the reaction, 1% agarose gel electrophoresis was performed and the DNA fragment was isolated from the gel by electrophoresis. 100 pmole each of this DNA fragment and the chemically synthesized oligonucleotide indicated in Fig. 5 were mixed and ligated. The ligation mixture was used to transform Escherichia coli W3110 according to a commonly used method, and tetracycline-resistant transformants were obtained.

After analyzing the plasmid of the transformed strain by gel electrophoresis after cleavage with restriction enzymes, E. coli W3110/pG97S4DhCT(G)R1 strain, W3110/pG97S4DhCT(G)R3 strain, W3110/pG97S4DhCT(G)R4 strain and W3110/pG97S4DhCT(G)R5 strain were obtained.

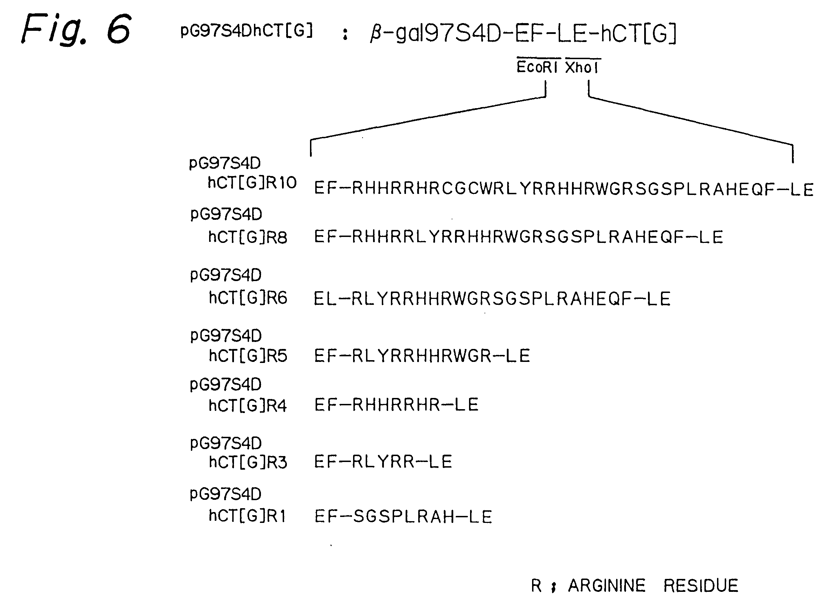

As indicated above, 7 types of plasmids were constructed wherein a portion of a tetracycline-resistant gene was inserted into the EcoRI cleavage site or EcoRI-XhoI cleavage site of pG97S4DhCT(G). As these inserted genes contain from 1 to a maximum of 10 codons corresponding to the basic amino acid arginine, the charges differ between the chimeric proteins thus resulting in the production of fusion proteins having different isoelectric points. The structures of the fusion proteins produced are indicated in Fig. 6.

### Example 2: Expression of hCT(G) Fusion Protein

In order to examine the relationship between the isoelectric points of fusion proteins produced in microorganisms and their productivity, the microorganisms were cultured and the productivity of fusion protein per number of cells was determined using SDS polyacrylamide gel electrophoresis.

The prepared microorganism was cultured in a flask for 12 hours at 37°C in 500 ml of SB medium (0.5% glycerine, 2.4% yeast extract, 1.2% tryptone, 100 mM potassium hydrogen phosphate pH 7.5 and tetracycline (10 µg/ml)). Following culturing, the turbidity of the culture broth was measured with a spectrophotometer (OD₆₆₀), and the culture broth was removed so that the value of [OD₆₆₀ value x culture volume (ml)] became 5, followed by centrifugation for 5 minutes at 12000 rpm to separate the microbial cells.

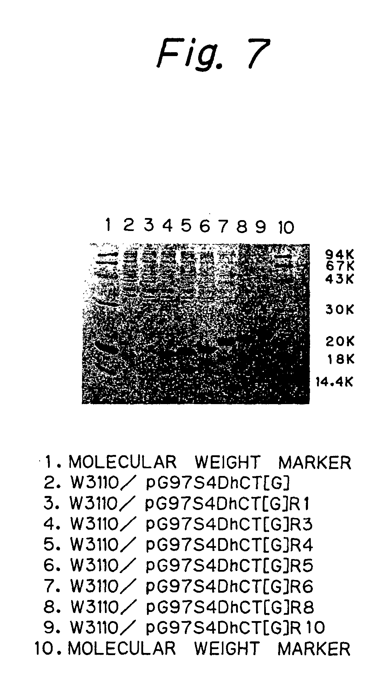

1 ml of SDS sample buffer (63 mM Tris-HCl pH 6.8, 10% glycerine, 10% SDS, 5% 2-mercaptoethanol and 12.5 mg/L bromphenol blue) was added to the cell precipitate followed by heating for 5 minutes at 95°C to obtain the sample for SDS polyacrylamide gel electrophoresis. SDS-16% polyacrylamide gel electrophoresis (TEFCO) was performed using 5 µl of the above-mentioned sample under the conditions of 18 mA for 90 minutes. Following the electrophoresis, the gel was dyed with a dye solution (10% acetic acid, 40% methanol and 2 g/L of Coomassie brilliant blue R-250) and the productivity per number of cells of the hCT(G) fusion protein produced by each strain were compared. The results of electrophoresis are indicated in Fig. 7.

As is clear from Fig. 7, strain W3110/pG97S4DhCT(G) providing an isoelectric point of 4.43 produced only an extremely small amount of fusion protein, while strain W3110/pG97S4DhCT(G)Rl providing an isoelectric point of 4.70 only produced a small amount of fusion protein. It was also clear that the other strains produced a large amount of fusion protein in cells. Thus, these results indicated that productivity of fusion protein as well as the amount of its inclusion bodies that are formed becomes poor when the isoelectric point of the fusion protein is in the vicinity of 4.5-4.7.

Next, a study was conducted on the productivity of fusion protein and the efficiency at which hCT(G) is released from fusion protein using V8 protease under conditions of large volume culturing by performing large volume culturing and production in 30 liter culturing tanks using strains W3110/pG97S4DhCT(G)R3, W3110/pG97S4DhCT(G)R4, W3110/pG97S4DhCT(G)R5, W3110/pG97S4DhCT(G)R6, W3110/pG97S4DhCT(G)R8 and W3110/pG97S4DhCT(G)R10.

### Example 3: Productivity of Fusion Protein Under Large Volume Culturing Conditions and Release of hCT(G) from Fusion Protein Using V8 Protease

The following experiment was carried out to study the productivity of fusion proteins under large volume culturing conditions and the efficiency of release of hCT(G) from various hCT(G) fusion proteins by V8 protease.

Six high expression strains (W3110/ pG97S4DhCT(G)R3, W3110/pG97S4DhCT(G)R4, W3110/ pG97S4DhCT(G)R5, W3110/pG97S4DhCT(G)R6, W3110/ pG97S4DhCT(G)R8 and W3110/pG97S4DhCT(G)R10), in which formation of inclusion bodies was observed in flask cultures using the above-mentioned SB medium, were cultured in 30 liter culturing tanks.

The medium contained 4 g/L of yeast extract, 4 g/L of potassium dihydrogen phosphate, 4 g/L of dipotassium hydrogen phosphate, 2.7 g/L of sodium hydrogen phosphate, 1.2 g/L of ammonium sulfate, 0.2 g/L of ammonium chloride, 2.0 g/L of L-methionine, 2.0 g/L of MgSO₄·7H₂O, 40 mg/L of FeSO₄·7H₂O, 40 mg/L of CaCl₂·2H₂O, 10 mg/L of AlCl₃·6H₂O, 4 mg/L of CoCl₂·6H₂O, 2 mg/L of ZnSO₄·7H₂O, 2 mg/L of Na₂MoO₄·2H₂O, 1.0 mg/L of CuCl₂·2H₂O, 0.5 mg/L of H₃BO₃ and 10 mg/L of MnSO₄·nH₂O, using glucose (2%) for the carbon source in the initial phase of culturing, and using glycerine (8%) as the carbon source after consumption of glucose. The results of the final attained bacterial concentration are indicated in Table 1.

**Table 1**

| Strain | Final Bacterial Concentration (OD₆₆₀) (a) | Quantity of Inclusion Bodies per Bacterium (Relative Value) (b) | Total Quantity of Inclusion Bodies (Relative Value) (a x b) |
|---|---|---|---|
| W3110/pG97S4Dh CT(G)R10 | 53 | 116 | 6148 |
| W3110/pG97S4Dh CT(G)R8 | 120 | 113 | 13560 |
| W3110/pG97S4Dh CT(G)R6 | 110 | 106 | 11660 |
| W3110/pG97S4Dh CT(G)R5 | 105 | 100 | 10500 |
| W3110/pG97S4Dh CT(G)R4 | 94 | 100 | 9400 |
| W3110/pG97S4Dh CT(G)R3 | 106 | 96 | 10176 |

As is clear from Table 1, strain W3110/pG97S4DhCT(G)R10 demonstrated remarkably poor growth in comparison to the other strains, reaching a final attained bacterial concentration roughly only half that of the other strains. In addition, although the isoelectric point of the fusion protein produced by strain W3110/pG97S4DhCT(G)R10 was slightly alkaline at 7.85, and there was an increase in the productivity of fusion protein per cell, perhaps due to the rapid formation of inclusion bodies or the isoelectric point of the fusion protein being slightly alkaline, cell growth was inhibited which resulted in a low cell concentration.

Thus, based on the results of these studies, it was verified for the -first time by the inventors of the present invention that in order to produce fusion protein as inclusion bodies in Escherichia coli both stably and in large amounts, it is important to maintain the isoelectric point of the fusion protein in the acidic region between 4.9 and 6.9. In addition, following the SDS polyacrylamide gel electrophoresis and dyeing proteins, the amount of fusion protein produced per cells was determined with a gel scanner. Those results are indicated in Table 1 as the amount of inclusion bodies per cells (relative value).

When the amount of fusion protein per cells was taken to be 100 for strain W3110/pG97S4DhCT(G)R4, each of the strains produced 96-116 inclusions. The total amount of inclusion bodies per culture liquid was 6,148 for strain W3110/pG97S4DhCT(G)R10 strain and 13560 for strain W3110/pG97S4DhCT(G)R8, indicating a difference of nearly a factor of 2. Although strain W3110/pG97S4DhCT(G)R8 demonstrated the largest total amount of inclusion bodies per culture broth, a cleavage reaction took place by V8 protease from fusion protein during production of hCT, with the efficiency of the cleavage reaction by V8 protease from fusion protein being closely involved with the yield of the production process.

As such, a study was conducted of the efficiency of the cleavage reaction by V8 protease using the inclusion bodies obtained. After culturing using the above-mentioned 30 liter culturing tanks, 1000 ml of culture broth was removed followed by homogenization of the cells using a high-pressure homogenizer (Manton Gaulin Laboratory Homogenizer 15M-8TA) at 600 Kg/cm². The precipitate containing the inclusion bodies was collected by centrifugation for 30 minutes at 7000 rpm. After addition of deionized water to the precipitate fraction to make the amount of the fraction equal to the initial volume, the suspension was centrifuged again to wash the precipitate.

After repeating the washing procedure one more time, the finally obtained precipitate was suspended with deionized water so that the OD₆₆₀ value became 800, and used in V8 protease cleavage reaction as described below. After removing 0.6 ml of suspension, adding 75 µl of 1 M Tris-HCl (pH 8.0), 540 mg of urea and 185 µl of 100 mM DTT to this suspension, and allowing to stand for 10 minutes at 30°C, deionized water was added to bring the final volume to 3.7 ml. After preheating for 10 minutes at 30°C, 7 µl of V8 protease (1 mg/ml) was added and allowed to react for 1 hour.

Quantitative determination of the cleaved hCT(G) was performed by high performance liquid chromatography (HPLC) using a YMC Packed column A-302 (0.46 cm x 15 cm, Yamamura Chemical Research). Elution was performed with a linear concentration gradient using 0.1% trifluoroacetic acid (TFA) and 0.1% TFA/50% acetonitrile. As a result, it was verified that the efficiency of cleavage of hCT(G) from fusion protein differed greatly depending on the hCT(G) fusion protein.

Strain W3110/pG97S4DhCT(G)R4 demonstrated the highest cleavage efficiency of 97%, while strain W3110/pG97S4DhCT(G)R5 demonstrated the lowest cleavage efficiency of 7%. There was no correlation whatsoever between this cleavage efficiency and the final bacterial concentration. In addition, there was also no correlation observed for the number of basic amino acid residues of the linker peptide inserted into the fusion protein. On the contrary, the cleavage efficiency was believed to be influenced by the amino acid sequence of the region of the cleavage recognition site for V8 protease. In any case, as strain W1110/pG97S4Dh CT(G)R4 demonstrated the largest amount of recovery of hCT(G), conversion from human calcitonin precursor to human calcitonin by an amidation enzyme was performed using this bacterial strain.

### Example 4: Purification of hCT(G) Precursor and Conversion to Human Calcitonin by an Amidation Enzyme

After culturing strain W3110/pG97S4DhCT(G)R4 in a 20 liter culturing tank in the manner described above, a suspension of inclusion bodies of fusion protein was obtained according to the method described above. After removing 0.6 ml of this suspension of inclusion bodies, adding 750 µl of 1 M Tris-HCl (pH 8.0), 75 µl of 0.5 M EDTA (pH 8.0), 5.4 g of urea and 17 mg of DTT, and allowing to stand for 10 minutes, deionized water was added to bring the final volume to 37 ml. Next, 40 µl of V8 protease (1 mg/ml) was added to treat the suspension for 90 minutes at 37°C.

After that diluting the reaction solution by a factor of 2 with deionized water, acetic acid was added after allowing to stand for 30 minutes to bring the pH to 4.6. The β-gal97S4D of the protective peptide precipitated as a result of lowering the pH to 4.6 while hCT(G) remained in the supernatant fraction. The supernatant fraction was separated by centrifuging for 15 minutes after which this supernatant fraction was applied to a column of SP Sepharose (Tosoh) equilibrated with 10 mM ammonium acetate (pH 4.6) followed by column chromatography. hCT(G) eluted in stages with 40 mM ammonium acetate (pH 6.5). Roughly 0.4 g of hCT(G) were obtained per liter of culture liquid.

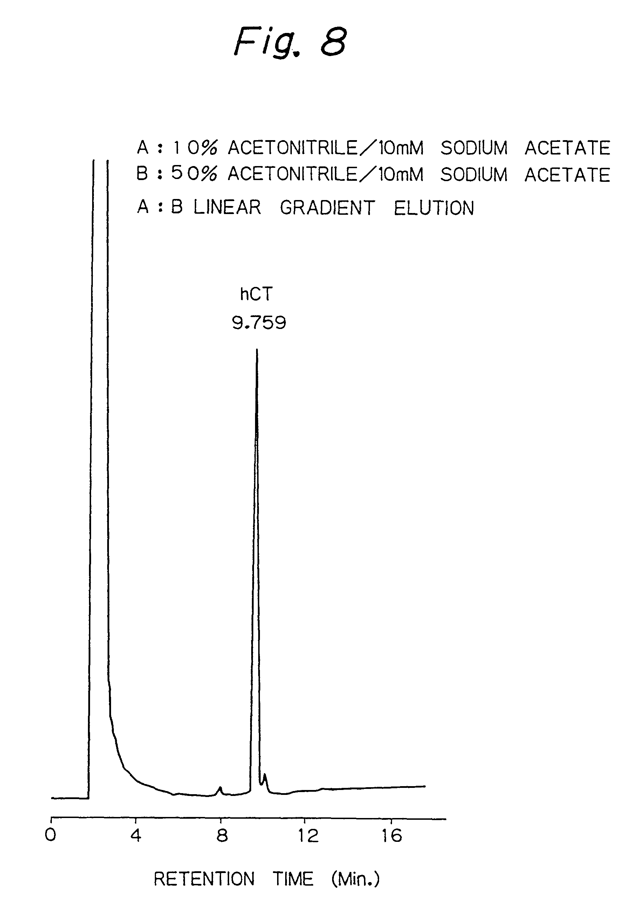

Human calcitonin was able to be produced with good efficiency by reacting the hCT(G) obtained in the manner above with amidation enzyme according to the method described in JP-A-2/190193.
Following the amidation reaction, high performance liquid chromatography was performed using YMC packed column A-302 (Yamamura Chemical Research), the results of which are indicated in Fig. 8. Under these elution conditions, human calcitonin eluted at a retention time of 9.7 minutes, and as is clear from this figure, it is possible to obtain human calcitonin having a high purity in extremely high yield.

### Example 5: Preparation of Human CNP-22 Gene

A gene coding for human CNP-22, in which a glutamic acid residue, the severing site of V8 protease, is added to the N terminal, was prepared as indicated below using in vitro DNA amplification (PCR).

pUCCNPl plasmid was used for the template DNA. 1.57 µg of pUCCNPl was digested for 60 minutes at 37°C in 157 µl of K buffer (20 mM Tris/HCl pH 8.5, 100 mM KCl and 1 mM dithreitol abbreviated as DTT) with 12 units of EcoRI to cleave the plasmid. Phenol treatment, 2-butanol treatment and ethanol precipitation were performed on the reaction solution according to commonly used methods, and EcoRI-digested DNA fragment was dissolved in 157 µl of TE buffer (10 mM Tris/HCl pH 8.0 and 1 mM EDTA).

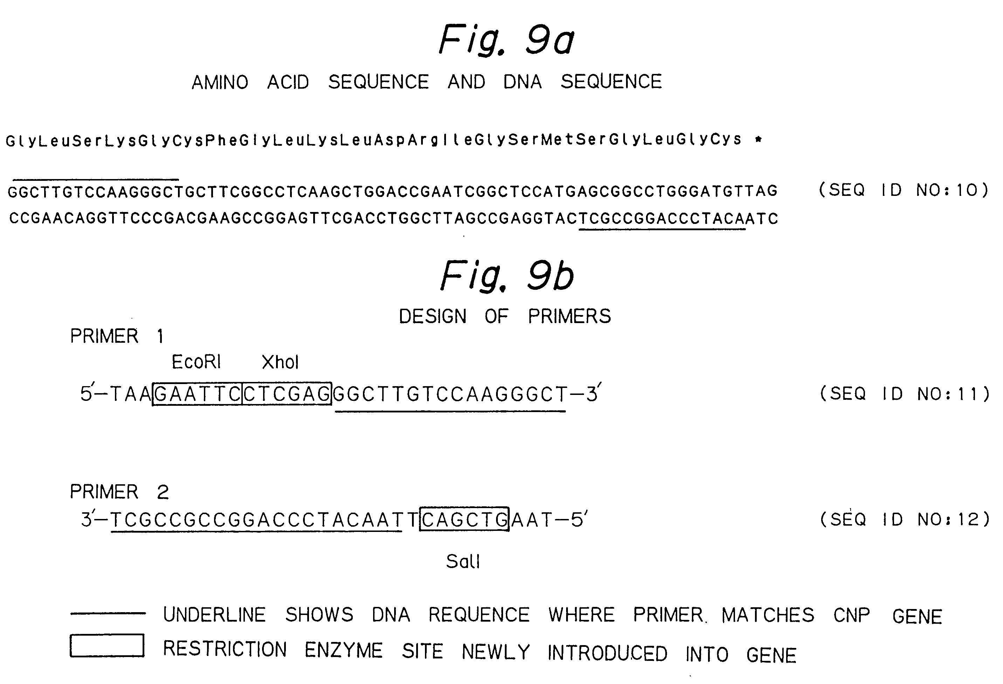

The designs of the primers used for the PCR reaction are indicated in Fig. 9. Primer 1 (SEQ ID NO 11) was designed so that a glutamic acid residue that is cleaved by V8 protease is added to the N terminal of human CNP-22, and EcoRI and XhoI restriction enzyme cleavage sites are provided further upstream, while primer 2 (SEQ ID NO 12) was designed so that a Sall restriction enzyme cleavage site is provided immediately after the human CNP-22 gene. These primers were synthesized using a DNA synthesizer (Applied Biosystems, Model 380A). Following synthesis, electrophoresis was performed using 20% polyacrylamide gel containing 8 M urea and the DNA fragments of a length corresponding to the primers were released to produce each of the primers.

After allowing 10 ng of pUCCNPl cleaved with the above-mentioned EcoRI and 100 µl of reaction solution (10 mM Tris/HCl pH 8.3, 50 mM KCl, 1.5 mM MgCl₂, 0.1% gelatin and 200 µM each of dGTP, dATP, dTTP and dCTP) containing primer (1 pmol each) to stand for 5 minutes at 95°C, the solutions were rapidly cooled with ice. 0.5 units of Taq DNA polymerase (Ampli-Taq, Takara Shuzo) were then added followed by the addition of mineral oil to carry out the PCR reaction using a thermal reactor (Hybaid).

The PCR reaction was repeated for 30 cycles, with a single cycle consisting of consecutive reactions consisting of thermal denaturation (92°C, 1 minute), annealing (55°C, 2 minutes) and DNA elongation (72°C, 3 minutes). DNA elongation reaction of the final cycle was further performed for an additional 7 minutes. Following the reaction, after addition of TE buffer to bring to a volume of 400 µl, the entire volume was placed in a SUPREC-02 (Takara Shuzo) and centrifuged for 8 minutes at 2,000 G. The filtrate was removed and TE buffer was again added to bring the amount of liquid up to 400 µl. The centrifugation procedure was then performed again in the same manner. The PCR reaction solution remaining in the filter cup was brought to a volume of 40 µl with TE buffer.

5 µl of 10 times-concentrated High buffer (100 mM Tris/HCl pH 7.5, 1 M NaCl, 100 mM MgCl₂ and 10 mM DTT), 36 units of EcoRI and 60 units of Sall were added to the 40 µl of PCR reaction mixture. After bringing the total volume to 50 µl with water, the reaction was allowed to proceed for 60 minutes at 37°C. Following the reaction, phenol treatment, 2-butanol treatment and ethanol precipitation were performed, and the precipitate was finally dissolved in TE buffer to prepare the human CNP-22 gene having EcoRI and Sall cohesive ends on both ends of the gene.

### Example 6-(a): Construction of Expression Plasmid pG97S4DhCNP-22

The PCR product of the CNP-22 gene indicated in Fig. 10 was inserted into plasmid pG97S4DhCT(GRRR) containing the β-gal97S4D (peptide consisting of 97 amino acids of the N-terminal of β-galactosidase, in which a cysteine residue is substituted by a serine residue, and four glutamic acid residues are substituted by aspartic acid residues) gene. That construction process is described below.

10 µg of pG97S4DhCT(GRRR) was digested with 36 units of EcoRI and 60 units of Sail in 70 µl of High buffer for 6 minutes at 37°C. Following the reaction, 1.0% agarose gel electrophoresis was performed according to commonly used methods, and a band of 3.2 Kb EcoRI-SalI fragment was cut out from the gel. The DNA fragment in the gel slice was extracted using a SUPREC-01 (Takara Shuzo) microcentrifuge tube followed by purification by phenol treatment, chloroform treatment and ethanol precipitation.

Next, 5 µl each of this EcoRI-Sall DNA fragment and the PCR product human CNP-22 gene fragment (EcoRI-Sall DNA fragment) were mixed, and ligated with a DNA ligation kit (Takara Shuzo). The ligation mixture was used to transform Escherichia coli W3110, and one of the transformants was named W3110/pG97S4DhCNP-22 strain.

### Example 6-(b): Construction of Expression PlasmidpG97S4DhCNP-22R3-2 and pG97S4DhCNP-22R5-2

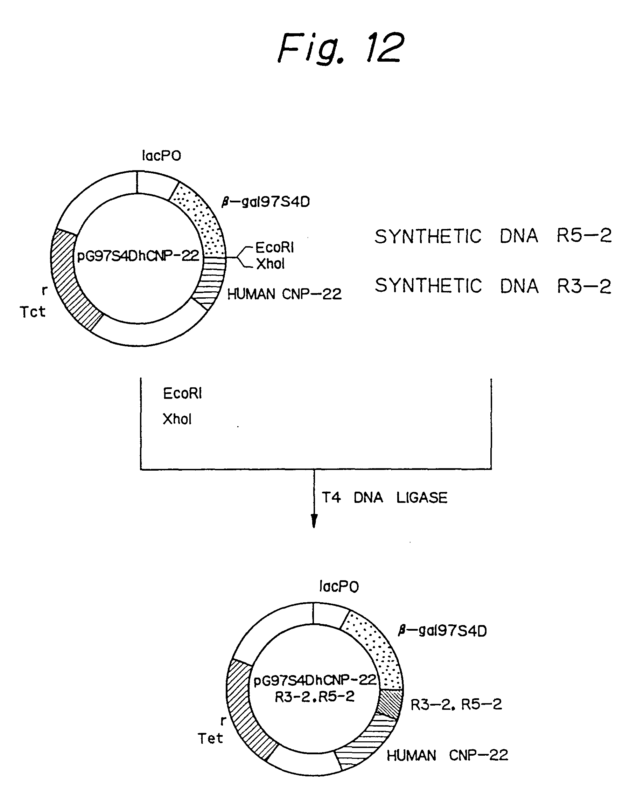

The following procedure was performed to insert a linker peptide gene coding for the amino acid sequence of R3-2 or R5-2 indicated in Fig. 11 into the linker peptide-coding region between β-gal197S4D gene and human CNP-22 gene. The construction of pG97S4DhCNP-22R3-2 and pG97S4DhCNP-22R5-2 is indicated in Fig. 12.

3 µg of pG97S4DhCNP-22 was digested with 10 units each of EcoRI and XhoI for 2 hours at 37°C in 50 µl of High buffer. Following the reaction, 1% agarose gel electrophoresis was performed and a 3.2 kb DNA fragment was isolated from the gel by electroelution. Ligation was performed on this DNA fragment and 20 pmol of chemically synthesized oligonucleotide coding for the R3-2 and R5-2 sequences using a ligation kit (Takara Shuzo). The ligation mixture was used to transform Escherichia coli W3110. Following isolation of the plasmid from the tetracycline-resistant transformed strain, structural analysis of the plasmid was performed using restriction enzymes to obtain the target W3110/pG97S4DhCNP-22R3-2 and W3110/pG97S4DhCNP-22R5-2.

### Example 6-(c): Construction of pG97S4DhCNP-22R5-3

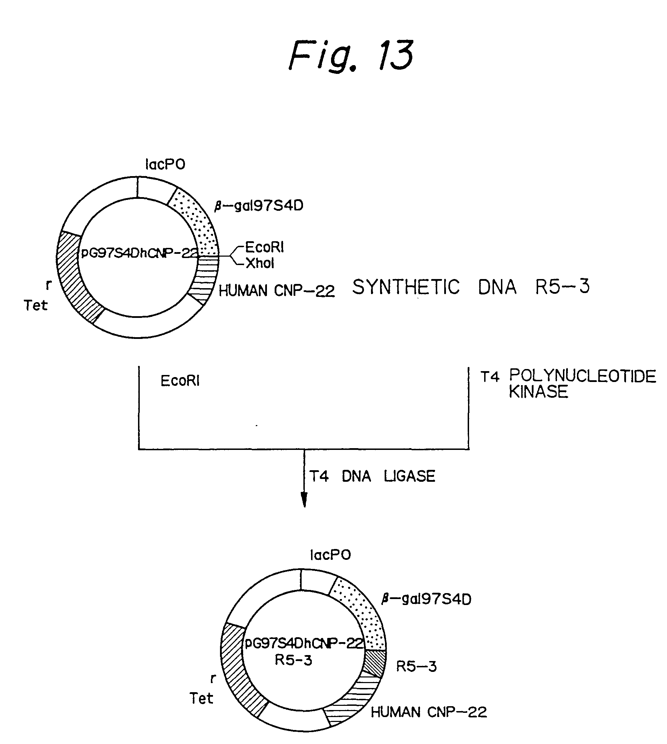

The following procedure was performed to insert a linker peptide gene coding for the amino acid sequence of R5-3 indicated in Fig. 11 into the pG97S4DhCNP-22 plasmid. The construction of pG97S4DhCNP-22R5-3 is indicated in Fig. 13.

3 µg of pG97S4DhCNP-22 was digested with 10 units of EcoRI for 2 hours at 37°C in 50 µl of High buffer. After digestion, 0.5 units of alkaline phosphatase was added to dephosphorylate the 5' terminal for 1 hour at 37°C. And then, phenol treatment was performed to inactivate alkaline phosphatase. 1% agarose gel electrophoresis was carried out, and the 3.2 Kb DNA fragment was isolated from the gel by electrophoresis. Next, 3 µg of chemically synthesized oligonucleotide coding for the amino acid sequence of R5-3 was treated with 20 units of T4 polynucleotide kinase in 100 µl of T4 polynucleotide kinase reaction solution (50 mM Tris/HCl pH 8.0, 10 mM MgCl₂, 10 mM 2-mercaptoethanol and 1 mM ATP) to phosphorylate the 5' end for 1 hour at 37°C.

Ligation was then performed on 2 µl of this phosphorylated oligonucleotide and the above-mentioned EcoRI-cleaved, alkaline phosphatase-treated pG97S4DhCNP-22. The ligation mixture was used to transform Escherichia coli W3110, and tetracycline-resistant transformants were obtained. The plasmids were analyzed by restriction enzymes, and W3110/pG97S4DhCNP-22R5-3 was obtained.

### Example 7: Expression of Human CNP-22 Fusion Protein

In order to examine the relationship between the isoelectric points of fusion proteins expressed in microbial cells and productivity, microorganisms were cultured and the productivity of fusion protein per number of cells was investigated using SDS polyacrylamide gel electrophoresis.

Escherichia coli strains were cultured in flasks for 12 hours at 37°C in 500 ml of Nul medium (0.4 g/L of yeast extract, 4 g/L of potassium dihydrogen phosphate, 4 g/L of dipotassium hydrogen phosphate, 2.7 g/L of disodium hydrogen phosphate, 1.2 g/L of ammonium sulfate, 0.2 g/L of ammonium chloride, 0.8% glycerine, 2 g/L of magnesium sulfate and 10 mg/L of tetracycline).

Following the culturing, the turbidity (OD₆₆₀) of the culture broth was measured with a spectrophotometer and culture broth was adjusted to the value of (OD₆₆₀ value x culture volume (ml)) became 5, and centrifugation was carried out for 5 minutes at 12000 rpm to separate the microbial cells. 1 ml of SDS sample buffer (63 mM Tris/HCl pH 6.8, 10% glycerine, 10% SDS, 5% 2-mercaptoethanol and 12.5 mg/L of bromphenol blue) was added to these cell precipitate and heated for 5 minutes at 95°C to obtain the sample for SDS polyacrylamide gel electrophoresis.

SDS-15% polyacrylamide gel electrophoresis (TEFCO) was performed using 5 µl of the above-mentioned sample under the conditions of 18 mA for 90 minutes. Following the electrophoresis, the gel was stained in a dye solution (10% acetic acid, 40% methanol and 2 g/L of Coomassie brilliant blue R-250) and the productivity per cell of human CNP-22 fusion protein produced in each strain was compared. The results of electrophoresis are indicated in Fig. 14.

As as indicated in Fig. 14, it is clear that when strain W3110/pG97S4DhCNP-22 providing an isoelectric point of 4.56 is compared with the other strains (W3110/pG97S4DhCNP-22R3-2 providing an isoelectric point of 4.95, W3110/pG97S4DhCNP-22R5-2 providing an isoelectric point of 6.22 and W3110/pG97S4DhCNP-22R5-3 providing an isoelectric point of 5.59), strains W3110/pG97S4DhCNP-22R3-2, W3110/pG97S4DhCNP-22R5-2 and W3110/pG97S4DhCNP-22R5-3 demonstrated greater productivity of fusion protein than strain W3110/pG97S4DhCNP-22.

It was indicated in particular that strains W3110/pG97S4DhCNP-22R5-2 and W3110/pG97S4DhCNP-22R5-3 expressed a large amount of fusion protein. Thus, it was verified in this Example as well that adjusting the isoelectric point of the fusion protein within the range of 4.9-6.9 results in increased productivity of fusion protein.

### Example 8: Release of Human CNP-22 from Fusion Protein by V8 Protease

The following experiment was conducted to examine the efficiency of release of human CNP-22 from fusion protein using V8 protease.

After culturing strains W3110/pG97S4DhCNP-22R3-2, W3110/pG97S4DhCNP-22R5-2 and W3110/pG97S4DhCNP-22R5-3 in the above-mentioned Nul medium, 400 ml of the culture broth was harvested and homogenized by a high-pressure homogenizer (Manton Gaulin Laboratory Homogenizer 15M-8TA) at 600 Kg/cm².

A precipitate containing the inclusion bodies was collected by centrifugation for 30 minutes at 7000 rpm. 400 ml of buffer A (50 mM Tris/HCl pH 8.0, 2 mM EDTA and 1% Triton X-100) was added to the resulting precipitate fraction, and the precipitate was washed by centrifuging again after suspending. This precipitation procedure was performed twice using buffer A and once using deionized water. After suspending the finally obtained precipitate in deionized water so that the OD₆₆₀ value became 20, the fusion protein was cleaved by V8 protease using the method indicated below.

6 µl of 1 M Tris/HCl (pH 8.0), 0.6 µl of 0.5 mM EDTA, 36 mg of urea and 3 µl of 1M DTT were added to 60 µl of the inclusion body suspension and then allowed to stand for 10 minutes. After standing, deionized water was added to bring the final volume to 300 µl. 1 µl of V8 protease (1 mg/ml) was then added and a cleavage reaction was allowed to proceed for 1 hour at 30°C. Quantitative determination of the human CNP-22 cleaved from the fusion protein was performed by high performance liquid chromatography (HPLC) using a YMC Packed column A-302 (2.46 cm x 15 cm, Yamamura Chemical Research).

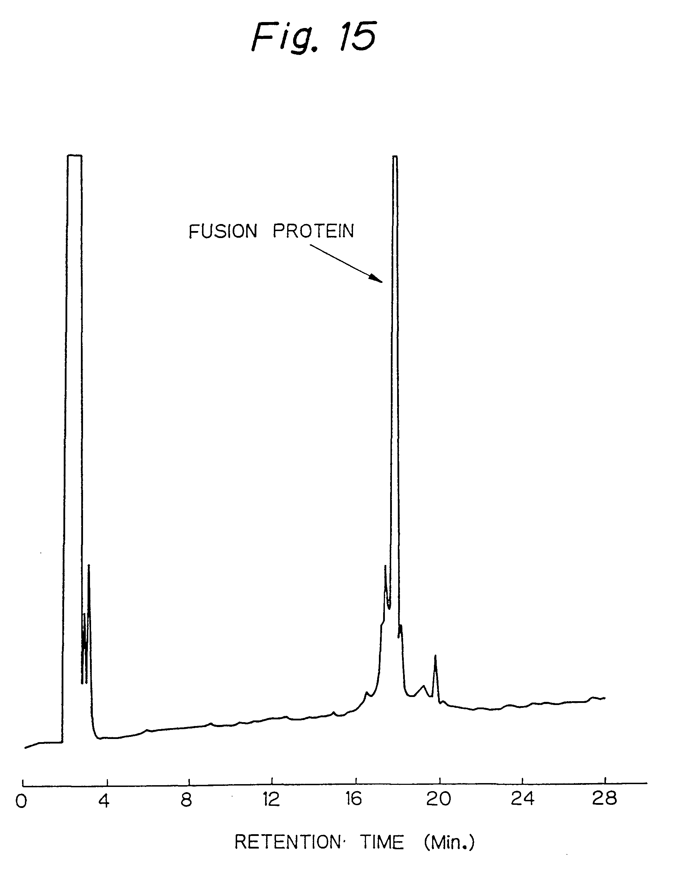

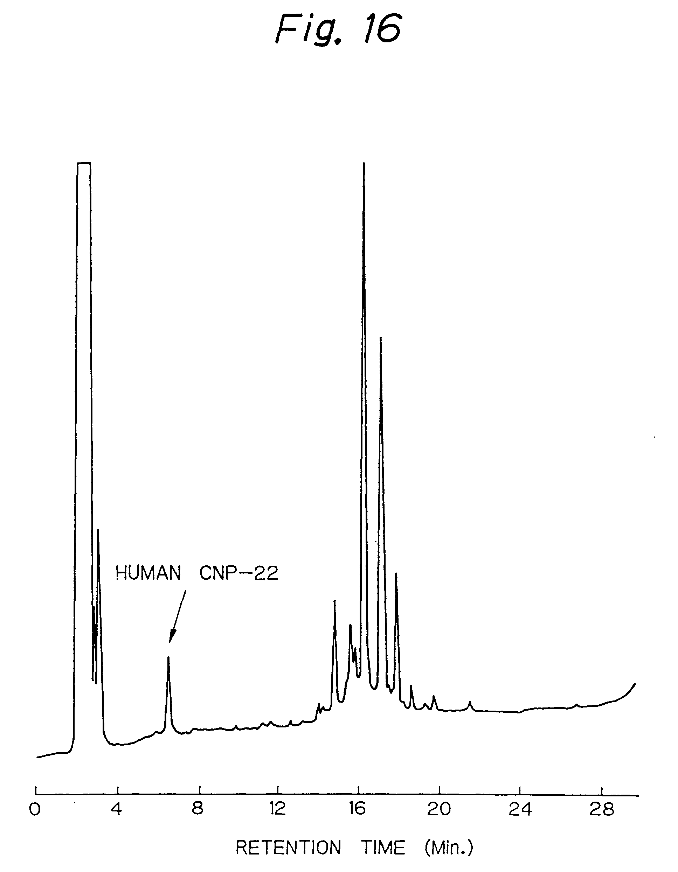

The V8 protease reaction solution was diluted by a factor of 20 with 2 M urea and 6% acetic acid solution followed by HPLC analysis using 20 µl of that diluted solution. HPLC elution was performed with a linear concentration gradient using 0.1% trifluoroacetic acid (TFA), 0.1% TFA and 50% acetonitrile. The elution patterns before and after cleavage of β-ga197S4DhCNP-22R5-3 fusion protein by V8 protease are indicated in Fig. 15 and Fig. 16, respectively. As is clear from Figs. 15 and 16, a peak appeared from the V8 protease-digested fusion protein was coincident with that of the human CNP-22 standard, thus indicating that human CNP-22 is specifically released from the fusion protein.

Release of human CNP-22 also took place efficiently in β-gal97S4DhCNP-22R5-2 and β-gal97S4DhCNP-22R3-2, with peaks being identified that matched the human CNP-22 standard. The release efficiency of the human CNP-22 cleaved under the above-mentioned conditions was 99% for pG97S4DhCNP-22R3-2, 95% for pG97S4DhCNP-22R5-2 and 92% for pG97S4DhCNP-22R5-3.

Based on the results described above, it was verified in not only the example of human calcitonin previously reported by the inventors of the present invention, but also in this Example as well, that in the production of human CNP-22, human CNP-22 can be produced in a large amount by altering the charge of the amino acids of the linker peptide region so that the isoelectric point of the fusion protein lies within the range of 4.9-6.9, and also that CNP-22 is specifically released from fusion protein produced in a large amount in the above manner by using V8 protease.

In the present work, it was verified for the first time by the present inventors that fusion protein may be produced in a large amounts in the form of inclusion bodies within microbial cells by designing the fusion protein so that its isoelectric point is on the acidic side, thereby allowing the production of a large amount of fusion protein containing the target peptide.

Thus, a high level of productivity of fusion protein containing the target protein can be obtained by large volume culturing using host cells obtained in the present method, thereby allowing this to be adequately used in the production of physiologically active peptides on an industrial scale.

In addition, a method was established wherein following large volume culturing using host cells obtained using the present technique, precursor human calcitonin or human CNP-22 is released and purified from the fusion protein. Furthermore, it was demonstrated that the precursor human calcitonin was converted to the mature calcitonin in large scale, by using an amidating enzyme.

### SEQUENCE LISTING

SEQ ID NO: 1
   SEQUENCE LENGTH: 99
   SEQUENCE TYPE: nucleic acid with corresponding protein
   STRANDNES: double
   TOPOLOGY: linear
   MOLECULE TYPE:
   SORCE: plasmid pBR322
   SEQUENCE 
CGG CAT CAC CGG CGC CAC AGG TGC GGT TGC TGG CGC CTA TAT CGC 45
Arg His His Arg Arg His Arg Cys Gly Cys Trp Arg Leu Tys Arg
5 10 15
CGA CAT CAC CGA TGG GGA AGA TCG GGC TCG CCA CTT CGG GCT CAT 90
Arg His His Arg Trp Gly Arg Ser Gly Ser Pro Leu Arg Ala His
20 25 30
GAG CAA TTC 99
Glu Gln Phe
SEQ ID NO: 2
   SEQUENCE LENGTH: 30
   SEQUENCE TYPE: nucleic acid
   STRANDNES:single
   TOPOLOGY: linear
   MOLECULE TYPE: synthetic DNA
   SEQUENCE AATTCTCGGG CTCGCCACTT CGGGCTCATC 30
SEQ ID NO: 3
   SEQUENCE LENGTH: 30
   SEQUENCE TYPE: nucleic acid
   STRANDNES: single
   TOPOLOGY: linear
   MOLECULE TYPE: synthetic DNA
   SEQUENCE TCGAGATGAG CCCGAAGTGG CGAGCCCGAG 30
SEQ ID NO: 4
   SEQUENCE LENGTH: 21
   SEQUENCE TYPE: nucleic acid
   STRANDNES: single
   TOPOLOGY: linear
   MOLECULE TYPE: synthetic DNA
   SEQUENCE AATTCCGCCT ATATCGCCGA C 21
SEQ ID NO: 5
   SEQUENCE LENG 21:
   SEQUENCE TYPE: nucleic acid
   STRANDNES: single
   TOPOLOGY: linear
   MOLECULE TYPE: synthetic DNA
   SEQUENCE TCGAGTCGGC GATATAGGCG G 21
SEQ ID NO: 6
   SEQUENCE LENGTH: 27
   SEQUENCE TYPE: nucleic acid
   STRANDNES:single
   TOPOLOGY:linear
   MOLECULE TYPE: synthetic DNA
   SEQUENCE AATTCCGGCA TCACCGGCGC CACAGGC 27
SEQ ID NO: 7
   SEQUENCE LENGTH: 27
   SEQUENCE TYPE: nucleic acid
   STRANDNES: single
   TOPOLOGY: linear
   MOLECULE TYPE: synthetic DNA
   SEQUENCE TCGAGCCTGT GGCGCCGGTG ATGCCGG 27
SEQ ID NO: 8
   SEQUENCE LENGTH: 39
   SEQUENCE TYPE: nucleic acid
   STRANDNES: single
   TOPOLOGY: linear
   MOLECULE TYPE: synthetic DNA
   SEQUENCE AATTCCGCCT ATATCGCCGA CATCACCGAT GGGGAAGAC 39
SEQ ID NO: 9
   SEQUENCE LENGTH: 39
   SEQUENCE TYPE: nucleic acid
   STRANDNES: single
   TOPOLOGY: linear
   MOLECULE TYPE: synthetic DNA
   SEQUENCE TCGAGTCTTC CCCATCGGTG ATGTCGGCGA TATAGGCGG 39
SEQ ID NO: 10
   SEQUENCE LENGTH: 69
   SEQUENCE TYPE: nucleic acid with corresponding protein
   STRANDNES: double
   TOPOLOGY: linear
   MOLECULE TYPE:
   SOURCE: plasmid pUCCNP1
   SEQUENCE 
GGC TTG TCC AAG GGC TGC TTC GGC CTC AAG CTG GAC CGA ATC GGC 45
Gly Leu Ser Lys Gly Cys Phe Gly Leu Lys Leu Asp Arg Ile Gly
                5                   10                  15
TCC ATG AGC GGC CTG GGA TGT TAG 69
Ser Met Ser Gly Leu Gly Cys
                20
SEQ ID NO: 11
   SEQUENCE LENGTH: 31
   SEQUENCE TYPE: nucleic acid
   STRANDNES: single
   TOPOLOGY: linear
   MOLECULE TYPE: synthetic DNA
   SEQUENCE TAAGAATTCC TCGAGGGCTT GTCCAAGGGC T 31
SEQ ID NO: 12
   SEQUENCE LENGTH: 30
   SEQUENCE TYPE: nucleic acid
   STRANDNES: single
   TOPOLOGY: linear
   MOLECULE TYPE: synthetic DNA
   SEQUENCE TAAGTCGACT TAACATCCCA GGCCGCCGCT 30
SEQ ID NO: 13
   SEQUENCE LENGTH: 21
   SEQUENCE TYPE: nucleic acid
   STRANDNES: single
   TOPOLOGY: linear
   MOLECULE TYPE: synthetic DNA
   SEQUENCE: AATTCCGGCG CCGAGAGTTC C 21
SEQ ID NO: 14
   SEQUENCE LENGTH: 21
   SEQUENCE TYPE: nucleic acid
   STRANDNES:single
   TOPOLOGY:linear
   MOLECULE TYPE: synthetic DNA
   SEQUENCE: TCGAGGAACT CTCGGCGCCG G 21
SEQ ID NO: 15
   SEQUENCE LENGTH: 36
   SEQUENCE TYPE: nucleic acid
   STRANDNES: single
   TOPOLOGY: linear
   MOLECULE TYPE: synthetic DNA
   SEQUENCE: AATTCCGGCG CCATCACCGG CGCCACCGAG AGTTCC 36
SEQ ID NO: 16
   SEQUENCE LENGTH: 36
   SEQUENCE TYPE: nucleic acid
   STRANDNES: single
   TOPOLOGY: linear
   MOLECULE TYPE: synthetic DNA
   SEQUENCE TCGAGGAACT CTCGGTGGCG CCGGTGATGG CGCCGG 36
SEQ ID NO: 17
   SEQUENCE LENGTH: 21
   SEQUENCE TYPE: nucleic acid
   STRANDNES: single
   TOPOLOGY: linear
   MOLECULE TYPE: synthetic DNA
   SEQUENCE: AATTTCGACG CCGTCGCCGA G 21
SEQ ID NO: 18
   SEQUENCE LENGTH: 21
   SEQUENCE TYPE: nucleic acid
   STRANDNES: single
   TOPOLOGY: linear
   MOLECULE TYPE: synthetic DNA
   SEQUENCE

AATTCTCGGC GACGGCGTCG A 21

## Claims

1. A process for production of a target peptide selected from calcitonin, calcitonin precursor, natriuretic peptide (NP), cell growth factor and parathyroid hormone, comprising:
(a) culturing host cells transformed with vector able to express a gene coding for a fusion protein representable by the formula
A-L-B
in which
B is the target peptide;
A is a protective peptide, being a 90 to 210 amino acid N-terminal fragment of E.coli β-galactosidase, and
L is a linker peptide between the C-terminal of the protective peptide and the N-terminal of the target peptide,
the linker peptide L being a portion of the amino acid sequence shown in SEQ ID No:1 containing from 3 to 8 arginine residues, selected so that the target protein separates off when the fusion protein is treated with a predetermined chemical substance such as an enzyme, and giving the entire fusion protein an isoelectric point in the range 4.9-6.9;
(b) homogenising the cultured host cells to obtain an insoluble fraction comprising inclusion bodies;
(c) solubilizing the fusion protein in the inclusion bodies by treating the insoluble fraction with solubilizing agent; and,
(d) cleaving the peptide bond between the C-terminal amino acid residue of the linker and the N-terminal of the target peptide to release the target peptide.

2. A process according to claim 1 in which the linker peptide is coded by a part of the nucleotide sequence 403 to 495 of the pBR322 tetracycline-resistance gene.

3. A process according to claim 1 or 2 in which the target peptide is human calcitonin.

4. A process according to any one of claims 1 to 3 in which the target peptide is atrial natriuretic peptide, brain natriuretic peptide or C-type natriuretic peptide (CNP).

5. A process according to claim 4 in which the target peptide is CNP-22.

6. A process according to any one of claims 1 to 5 in which the host cells are E.coli.

7. A process according to any one of claims 1 to 6 in which the protective peptide consists of the N-terminal amino acids 1 to 97 of the β-galactosidase.

8. A process according to claim 7 in which, in said N-terminal β-galactosidase fragment protective peptide, serine residues replace the cysteine residues and aspartic acid residues replace four glutamic acid residues.

9. A process for production of a target peptide selected from calcitonin, calcitonin precursor, natriuretic peptide (NP), cell growth factor and parathyroid hormone, comprising:
(a) culturing host cells transformed with vector able to express a gene coding for a fusion protein representable by the formula
A-L-B
in which
B is the target peptide;
A is a protective peptide, being a 90 to 210 amino acid N-terminal fragment of E.coli β-galactosidase, and
L is a linker peptide between the C-terminal of the protective peptide and the N-terminal of the target peptide,
the linker peptide L being selected firstly so that the target protein separates off when the fusion protein is treated with a predetermined chemical substance such as an enzyme, and secondly so as to give the entire fusion protein an isoelectric point in the range 4.9-6.9;
(b) homogenising the cultured host cells to obtain an insoluble fraction comprising inclusion bodies;
(c) solubilizing the fusion protein in the inclusion bodies by treating the insoluble fraction with solubilizing agent, and
(d) cleaving the peptide bond between the C-terminal amino acid residue of the linker and the N-terminal of the target peptide to release the target peptide,
but excluding processes in which the linker peptide is a single lysine, arginine, glutamic acid or methionine residue, which is cleaved in step (d).

## Patentansprüche

1. Verfahren zur Herstellung eines Zielpeptids, ausgewählt aus Calcitonin, einer Calcitonin-Vorstufe, einem Natriuretikum-Peptid (NP), einem Zellwachstumsfaktor und einem Parathormon, welches umfaßt:
(a) Züchten von Wirtszellen, die mit einem Vektor transformiert sind, der ein Gen ausprägen kann, das ein Fusionsprotein codiert, das mit der Formel
A-L-B
angegeben werden kann, worin
B das Zielpeptid ist;
A ein Schutzpeptid ist, das ein N-terminales Fragment mit 90 bis 210 Aminosäuren von β-Galactosidase von E.coli ist; und
L ein Linker-Peptid zwischen dem C-Terminus des Schutzpeptids und dem N-Terminus des Zielpeptids ist; wobei das Linker-Peptid L ein Teil der in der Sequenzidentifizierungsnummer 1 gezeigten Aminosäuresequenz ist, die 3 bis 8 Argininreste enthält, das so ausgewählt ist, daß sich das Zielprotein abtrennt, wenn das Fusionsprotein mit einer bestimmten chemischen Substanz, wie einem Enzym, behandelt wird, und das gesamte Fusionsprotein einen isoelektrischen Punkt im Bereich von 4,9 bis 6,9 erhält;
(b) Homogenisieren der gezüchteten Wirtszellen, wodurch eine unlösliche Fraktion erhalten wird, die Einschlußkörper umfaßt;
(c) Lösen des Fusionsproteins in den Einschlußkörpern durch Behandeln der unlöslichen Fraktion mit einem löslichmachenden Mittel; und
(d) Spalten der Peptidbindung zwischen dem C-terminalen Aminosäurerest des Linkers und dem N-Terminus des Zielpeptids, wodurch das Zielpeptid freigesetzt wird.

2. Verfahren nach Anspruch 1, wobei das Linker-Peptid von einem Teil der Nucleotidsequenz 403 bis 495 des pBR322-Tetracyclin-Resistenzgens codiert wird.

3. Verfahren nach Anspruch 1 oder 2, wobei das Zielpeptid Human-Calciton ist.

4. Verfahren nach einem der Ansprüche 1 bis 3, wobei das Zielpeptid Natriuretikum-Peptid des Vorhofs, Natriuretikum-Peptid des Gehirns oder Natriuretikum-Peptid vom C-Typ (CNP) ist.

5. Verfahren nach Anspruch 4, wobei das Zielpeptid CNP-22 ist.

6. Verfahren nach einem der Ansprüche 1 bis 5, wobei die Wirtszellen E.coli sind.

7. Verfahren nach einem der Ansprüche 1 bis 6, wobei das Schutzpeptid aus den N-terminalen Aminosäuren 1 bis 97 von β-Galactosidase besteht.

8. Verfahren nach Anspruch 7, wobei in dem N-terminalen β-Galactosidasefragment-Schutzpeptid Serinreste die Cysteinreste ersetzen und Asparaginsäurereste vier Glutaminsäurereste ersetzen.

9. Verfahren zur Herstellung eines Zielpeptids, ausgewählt aus Calcitonin, einer Calcitonin-Vorstufe, einem Natriuretikum-Peptid (NP), einem Zellwachstumsfaktor und einem Parathormon, welches umfaßt:
(a) Züchten von Wirtszellen, die mit einem Vektor transformiert sind, der ein Gen ausprägen kann, das ein Fusionsprotein codiert, das mit der Formel
A-L-B
angegeben werden kann, worin
B das Zielpeptid ist;
A ein Schutzpeptid ist, das ein N-terminales Fragment mit 90 bis 210 Aminosäuren von β-Galactosidase von E.coli ist; und
L ein Linker-Peptid zwischen dem C-Terminus des Schutzpeptids und dem N-Terminus des Zielpeptids ist,
das Linker-Peptid L so ausgewählt wird, daß erstens sich das Zielprotein abtrennt, wenn das Fusionsprotein mit einer bestimmten chemischen Substanz, wie einem Enzym, behandelt wird, und zweitens das gesamte Fusionsprotein einen isoelektrischen Punkt im Bereich von 4,9 bis 6,9 erhält;
(b) Homogenisieren der gezüchteten Wirtszellen, wodurch eine unlösliche Fraktion erhalten wird, die Einschlußkörper umfaßt;
(c) Lösen des Fusionsproteins in den Einschlußkörpern durch Behandeln der unlöslichen Fraktion mit einem löslichmachenden Mittel; und
(d) Spalten der Peptidbindung zwischen dem C-terminalen Aminosäurerest des Linkers und dem N-Terminus des Zielpeptids, wodurch das Zielpeptid freigesetzt wird,
wobei jedoch Verfahren ausgenommen sind, bei denen das Linker-Peptid ein einzelnes Lysin, Arginin, Glutaminsäure oder ein Methioninrest ist, der im Schritt (d) abgespalten wird.

## Revendications

1. Un procédé de production d'un peptide cible choisi parmi la calcitonine, le précurseur de calcitonine, le peptide natriurétique (NP), les facteurs de croissance de cellules et hormone parathyroïde, comprenant :
(a) la culture de cellules hôtes transformées par des vecteurs susceptibles d'exprimer un gène codant pour une protéine de fusion représentée par la formule
A-L-B
dans laquelle
B est le peptide cible,
A est un peptide protecteur, consistant en un fragment N-terminal de 90 à 210 amino acide et de E. coli β-galactosidase, et L est un peptide lien entre la position C-terminale du peptide protecteur et la position N-terminale du peptide cible,
le peptide lien L étant une portion d'une séquence d'amino-acide définie dans SEQ ID N° est un peptide lien entre la position contenant de 3 à 8 résidus arginine et étant choisi de sorte que la protéine cible se clive lorsque la protéine de fusion est traitée par une substance chimique prédéterminée telle qu'une enzyme, et que la protéine de fusion en soi présente un point isoélectrique compris entre 4,9 et 6,9.
(b) on homogénéise les cellules hôtes cultivées pour obtenir une fraction insoluble comprenant des corps d'inclusion ;
(c) on solubilise la protéine de fusion dans les corps d'inclusion par traitement de la fraction insoluble avec un agent de solubilisation et
(d) on clive le lien peptide entre le résidu amino-acide C-terminal du lien et la position N-terminale du peptide cible pour libérer le peptide cible.

2. Un procédé selon la revendication 1, dans lequel le peptide lien est codé par une partie de la séquence nucléotide 403 à 495 du gène résistant à la tétracycline pBR322.

3. Un procédé selon la revendication 1 ou 2, dans lequel le peptide cible est la calcitonine humaine.

4. Un procédé selon une quelconque des revendications 1 à 3, dans lequel le peptide cible est un peptide natriurétique atriale, un peptide natriurétique de cervelle ou un peptide natriurétique de type C (CNP).

5. Un procédé selon la revendication 4, dans lequel le peptide cible est le CNP-22.

6. Un procédé selon l'une quelconque des revendications 1 à 5, dans lequel les cellules hôtes sont E.coli.

7. Un procédé selon l'une quelconque des revendications 1 à 6, dans lequel le peptide protecteur consiste en 1 à 97 acide aminés N-terminal, de la β-galactosidase.

8. Un procédé selon la revendication 7, dans laquelle, dans ledit fragment de β-galactosidase du peptide protecteur, des résidus de serine remplacent les résidus de cystéine et les résidus d'acide aspartique remplacent quatre résidus d'acide glutamique.

9. Un procédé de production d'un peptide cible choisi parmi la calcitonine, le précurseur de calcitonine, le peptide natriurégique (NP), les facteurs de croissance de cellules et l'hormone parathyroïde, comprenant :
(a) la culture de cellules hôtes transformées par des vecteurs susceptibles d'exprimer un gène codant pour une protéine de fusion représentée par la formule
A-L-B
dans laquelle
B est le peptide cible,
A est un peptide protecteur consistant en un fragment N-terminal de 90 à 210 amino acide de E.coli β-galactosidase, et L est un peptide lien entre la position C-terminale du peptide protecteur et la position N-terminale du peptide cible,
le peptide L-lien étant choisi tout d'abord de sorte que la protéine cible se détache lorsque la protéine de fusion est traitée par une substance chimique prédéterminée, telle qu'une enzyme, et deuxièmement que l'on obtienne pour la protéine de fusion en soi, un point isoélectrique compris entre 4,9 et 6,9.
(b) on homogénéise les cellules hôtes cultivées pour obtenir une fraction insoluble comprenant des corps d'inclusion ;
(c) on solubilise la protéine de fission dans les corps d'inclusion par traitement de la fraction insoluble avec un agent d'insolubilisation et
(d) on clive le lien peptide entre le résidu amino-acide C-terminal du lien et la position N-terminale du peptide cible pour libérer le peptide cible,
ce procédé excluant les procédés dans lesquels le peptide lien est un résidu de méthionine, d'acide glutamique, d'arginine ou de lysine qui est clivé dans l'étape (d).
